# EUROPÄISCHE PATENTANMELDUNG

(11) **EP 2 623 089 A2**
(43) Veröffentlichungstag der Anmeldung: **07.08.2013**
(21) Anmeldenummer: 12193797.3
(22) Anmeldetag: 22.11.2012
(51) Int. Cl.: A61K 8/31, A61K 8/37, A61K 8/58, A61K 8/81, A61K 8/898, A61K 8/92, A61Q 5/00, A61Q 5/12

(54) **Haarpflegemittel**

(30) Priorität: 08.12.2011 DE 102011087975; 08.12.2011 DE 102011087974; 08.12.2011 DE 102011087976
(71) Anmelder: Henkel AG&Co. KGAA, 40589 Düsseldorf (DE)
(72) Erfinder: Krueger, Marcus, 25373 Ellerhoop (DE); Battermann, Marlene, 21271 Asendorf (DE); Hippe, Thomas, 25482 Appen (DE); Delowsky, Jens, 22844 Norderstedt (DE)

(57) **Zusammenfassung**

Die Anmeldung beschreibt kosmetische Mittel auf Grundlage einer Wirkstoffkombination aus spezifischen Isoparaffinen und spezifischen Silikonen und/oder polymeren quartären Ammoniumverbindungen und/oder Ölkörpern sowie die Verwendung dieser Mittel zur Pflege keratinischer Fasern.

## Beschreibung

Die Anmeldung beschreibt kosmetische Mittel auf Grundlage einer Wirkstoffkombination aus spezifischen Isoparaffinen und spezifischen Silikonen und/oder polymeren quartären Ammoniumverbindungen und/oder Ölkörpern sowie die Verwendung dieser Mittel zur Pflege keratinischer Fasern.

Das menschliche Haar ist neben den natürlichen Umwelteinflüssen einer Reihe weiterer, insbesondere kosmetischer Beanspruchungen ausgesetzt. Zu diesen, das Haar strapazierenden Beanspruchungen zählen beispielsweise die Färbung des Haars sowie dessen Verformung, beispielsweise durch eine Dauerwelle.

Zur Minderung der nachteiligen Auswirkungen der die Haarstruktur verändernden (Umwelt)Einflüsse aber auch zur Erhaltung und Verbesserung der natürlichen Haarstruktur werden kosmetische Haarpflegemittel eingesetzt. Ein wesentlicher Wirkstoff in vielen dieser kosmetischen Mittel sind die Silicium-organischen Verbindungen, insbesondere die Silikone wie die Trisiloxane, die sich durch pflegende Eigenschaften auszeichnen. Die Nachteile dieser Trisiloxane sind die durch Benetzung der Haaroberfläche verminderte Penetration von Wirk- und Hilfsstoffen in das Haar und die ebenfalls durch die Benetzung der Haaroberfläche bedingte Erschwerung der Frisurgestaltung. Die Bereitstellung Trisiloxan armer oder Trisiloxan freier Pflegemittel ist daher eine relevante Aufgabe im Bereich der Haarkosmetik.

Als eine von zahlreichen zur Substitution der Silikone geeignete Wirkstoffklasse werden im Stand der Technik die Isoparaffine diskutiert. So beschreiben beispielsweise die europäischen Patente EP 413 417 B1 (Colgate Palmolive) und EP 1 284 712 B1 (Procter&Gamble) pflegende Haarshampoos bzw. Haarkonditionierer, die neben weiteren Bestandteilen auch Isoparaffine enthalten können. Diese Haarpflegemittel weisen jedoch weiterhin eine Reihe von Nachteilen auf und trotz des bisher Erreichten besteht weiterhin ein Bedarf an Trisiloxan armen oder Trisiloxan freien Haarpflegemitteln auf Isoparaffinbasis.

Es wurde nun festgestellt, dass durch die Kombination spezifischer Isoparaffine mit spezifischen Silikonen und/oder polymeren quartären Ammoniumverbindungen und/oder Ölkörpern Haarpflegemittel mit ausgezeichneter Pflegewirkung erhalten werden können. Diese Mittel bewirken gute Glanzeigenschaften, ein verbessertes Aussehen und eine verbesserte Regeneration der keratinischen Fasern.

Ein erster Gegenstand dieser Anmeldung ist daher ein kosmetisches Mittel, enthaltend in einem kosmetisch akzeptablen Träger
a) mindestens ein Isoparaffin aus der Gruppe Isodecan, Isoundecan, Isododecan, Isotridecan und Isotetradecan,
b) mindestens ein Polymer aus der Gruppe
   b1) der Silikonpolymere mit quartärer Ammoniumfunktion
   b2) der Silsesquioxane, und/oder
c) mindestens eine polymere quartäre Ammoniumverbindung und/oder
d) mindestens ein Ölkörper.

Die erfindungsgemäßen kosmetischen Mittel enthalten als ersten wesentlichen Bestandteil mindestens ein Isoparaffin aus der Gruppe Isodecan, Isoundecan, Isododecan, Isotridecan und Isotetradecan. Diese Isoparaffine zeichnen sich gegenüber den n-Paraffinen mit gleicher Anzahl an Kohlenstoffatomen bei Einsatz in den kosmetischen Mitteln durch verbesserte olfaktorische Eigenschaften aus. Gegenüber den n-Paraffinen mit gleicher Anzahl an Kohlenstoffatomen sowie gegenüber den Isoparaffinen mit geringerer oder höherer Anzahl an Kohlenstoffatomen wird in Kombination mit den Inhaltsstoffen b) und/oder c) und/oder d) zudem überraschend eine verbesserte kosmetische, insbesondere haarpflegende Wirkung erreicht.

Auf den Inhalt der Prioritätsdokumente DE 102011087975, DE 102011087974 und DE 102011087976 wird voll inhaltlich Bezug genommen und hingewiesen.

Als für die kosmetische Wirkung besonders vorteilhaft hat sich der Einsatz von Isoparaffinen a) aus der Gruppe Isoundecan, Isododecan und Isotridecan erwiesen. Entsprechende kosmetische Mittel, dadurch gekennzeichnet, dass sie mindestens ein Isoparaffin a) ausgewählt aus der Gruppe Isoundecan, Isododecan, Isotridecan enthalten, werden erfindungsgemäß bevorzugt. Besonders vorteilhafte Wirkungen in Kombination mit den weiteren Inhaltsstoffen b), mindestens einem Polymer aus der Gruppe b1) der Silikonpolymere mit quartärer Ammoniumfunktion, b2) der Silsesquioxane, und/oder c) mindestens einer polymeren quartären Ammoniumverbindung und/oder d) mindestens einem Ölkörper, zeigen erfindungsgemäße kosmetische Mittel, die als Isoparaffin a) eine Mischung aus Isoundecan, Isododecan und Isotridecan enthalten. In einer bevorzugten Ausführungsform sind erfindungsgemäße kosmetische Mittel daher dadurch gekennzeichnet, dass sie als Isoparaffin a) eine Mischung aus Isoundecan, Isododecan und Isotridecan, insbesondere eine Mischung aus Isododecan und Isotridecdan, enthalten.

Der Gewichtsanteil des Isoundecans, Isodedecans und Isotridecans am Gesamtgewicht aller in dem kosmetischen Mittel eingesetzten Isoparaffine beträgt vorzugsweise mehr als 62 Gew.-%, bevorzugt mehr als 72 Gew.-%, besonders bevorzugt mehr als 82 Gew.-% und insbesondere mehr als 92 Gew.-%. Besonders bevorzugt ist es, dass der Gewichtsanteil des Isodedecans und Isotridecans am Gesamtgewicht aller in dem kosmetischen Mittel eingesetzten Isoparaffine mehr als 60 Gew.-%, bevorzugt mehr als 70 Gew.-%, besonders bevorzugt mehr als 80 Gew.-% und insbesondere mehr als 90 Gew.-% beträgt.

Die Dichte bei 15°C (DIN 51 757 P.4; ASTM D 4052) bevorzugter Isoparaffingemische beträgt vorzugsweise 700 bis 820 kg/m³, bevorzugt 720 bis 800 kg/m³ und insbesondere 740 bis 780 kg/m³.

Bevorzugte Isoparaffingemische zeichnen sich durch einen Siedebereich (DIN ES ISO 3405; ASTM D 86) zwischen 195°C und 250°C, bevorzugt zwischen 200°C und 245°C und insbesondere zwischen 205° und 240°C aus. Ein erfindungsgemäß besonders bevorzugtes Isoparaffingemisch ist kommerziell unter der Bezeichnung Pionier^{®} 2094 (H&R Group) erhältlich. Der Gewichtsanteil des Isoparaffins a) am Gesamtgewicht des kosmetischen Mittels beträgt bevorzugt 5,0 bis 70 Gew.-%, vorzugsweise 8,0 bis 60 Gew.-% und insbesondere 10 bis 50 Gew.-%.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 1 | Formel 2 | Formel 3 | Formel 4 |
|---|---|---|---|---|
| Isoparaffin * | 5,0 bis 70 | 8,0 bis 60 | 10 bis 50 | 15 bis 40 |
| Polymer **, Misc | add 100 | add 100 | add 100 | add 100 |

| | Formel 5 | Formel 6 | Formel 7 | Formel 8 |
|---|---|---|---|---|
| Isoparaffin * | 5,0 bis 70 | 8,0 bis 60 | 10 bis 50 | 15 bis 40 |
| Polymer ***, Misc | add 100 | add 100 | add 100 | add 100 |

| | Formel 9 | Formel 10 | Formel 11 | Formel 12 |
|---|---|---|---|---|
| Isoparaffin * | 5,0 bis 70 | 8,0 bis 60 | 10 bis 50 | 15 bis 40 |
| Polymer **, Misc | add 100 | add 100 | add 100 | add 100 |

| | Formel 13 | Formel 14 | Formel 15 | Formel 16 |
|---|---|---|---|---|
| Isoparaffin * | 5,0 bis 70 | 8,0 bis 60 | 10 bis 50 | 15 bis 40 |
| Polymer ***, Misc | add 100 | add 100 | add 100 | add 100 |

| | | | | |
|---|---|---|---|---|
| * Isoparaffingemisch, umfassend mindestens ein Isoparaffin aus der Gruppe Isoundecan, Isododecan und Isotridecan, wobei der Gewichtsanteil des Isoundecans, Isodedecans und Isotridecans am Gesamtgewicht aller in dem kosmetischen Mittel eingesetzten Isoparaffine vorzugsweise mehr als 62 Gew.-%, bevorzugt mehr als 72 Gew.-%, besonders bevorzugt mehr als 82 Gew.-% und insbesondere mehr als 92 Gew.-% beträgt. ** Polymer aus der Gruppe der Silikonpolymere mit quartärer Ammoniumfunktion *** Polymer aus der Gruppe der Silsesquioxane * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. ** Polymer aus der Gruppe der Silikonpolymere mit quartärer Ammoniumfunktion *** Polymer aus der Gruppe der Silsesquioxane | | | | |

Als zweiten wesentlichen Bestandteil enthalten die erfindungsgemäßen kosmetischen Mittel mindestens ein Polymer aus der Gruppe
b1) der Silikonpolymere mit quartärer Ammoniumfunktion
b2) der Silsesquioxane.

Geeignete Polymere b1) sind ausgewählt aus der Gruppe der quartären Polydimethylsiloxane. Beispiele für die bevorzugten Silikonpolymere b1) sind insbesondere die Verbindungen mit den INCI-Bezeichnungen Silicone Quaternium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-17, Silicone Quaternium-18, Silicone Quaternium-20, Silicone Quaternium-21, Silicone Quaternium-22 sowie Silicone Quaternium-2 Panthenol Succinate und Silicone Quaternium-16/Glycidyl Dimethicone Crosspolymer. Der Gewichtsanteil des Silikonpolymers b1) am Gesamtgewicht des kosmetischen Mittels beträgt vorzugsweise 0,05 bis 5,0 Gew.-%, bevorzugt 0,1 bis 3,0 Gew.-%, besonders bevorzugt 0,2 bis 2,0 Gew.-% und insbesondere 0,5 bis 1,5 Gew.-%.

Bevorzugte Silikonpolymer b1) werden ausgewählt aus den Silikonpolymeren, die neben einer quartären Ammoniumfunktion weiterhin auch freie Hydroxylgruppen aufweisen.

Zur Gruppe der bevorzugten quartären Polydimethylsiloxane zählen beispielsweise diquartäre Verbindungen der allgemeinen Formel (Si3c)

[R¹R²R³N⁺-A-SiR⁷R⁸-(O-SiR⁹R¹⁰)ₙ-O-SiR¹¹R¹²-A-N⁺R⁴R⁵R⁶]2X⁻ (Si3c),

wobei
- die Reste R1 bis R6 unabhängig voneinander C1- bis C22-Alkylreste bedeuten, welche Hydroxygruppen enthalten können und wobei vorzugsweise mindestens einer der Reste mindestens 8 C-Atome aufweist und die übrigen Reste 1 bis 4 C-Atome aufweisen;
- die Reste R7 bis R12 unabhängig voneinander gleich oder verschieden sind und C1- bis C10-Alkyl oder Phenyl bedeuten;
- A eine divalente organische Verbindungsgruppe bedeutet;
- n eine Zahl von 0 bis 200, vorzugsweise von 10 bis 120, besonders bevorzugt von 10 bis 40 ist, und X⁻ ein Anion ist.

Die divalente Verbindungsgruppe ist vorzugsweise eine C1- bis C12-Alkylen-oder Alkoxyalkylengruppe, die mit einer oder mehreren Hydroxylgruppen substituiert sein kann. Besonders bevorzugt ist die Gruppe -(CH₂)₃-O-CH₂-CH(OH)-CH₂-.

Das Anion X⁻ kann ein Halogenidion, ein Acetat, ein organisches Carboxylat oder eine Verbindung der allgemeinen Formel RSO₃⁻ sein, worin R die Bedeutung von C1- bis C4-Alkylresten hat.

Ein bevorzugtes diquartäres Silikon hat die allgemeine Formel (Si3d)

[RN⁺Me₂-A-(SiMe₂O)ₙ-SiMe₂-A-N⁺Me₂R]₂CH₃COO⁻ (Si3d),

wobei
- A die Gruppe -(CH₂)₃-O-CH₂-CH(OH)-CH₂- ist;
- R ein Alkylrest mit mindestens 8 C-Atomen;
- n für eine Zahl von 10 bis 120 steht.

Geeignete Silikonpolymere mit zwei endständigen, quartären Ammoniumgruppen sind unter der INCI-Bezeichnung Quaternium-80 bekannt. Hierbei handelt es sich um Dimethylsiloxane mit zwei endständigen Trialkylammoniumgruppen. Derartige diquartäre Polydimethylsiloxane werden von der Firma Evonik unter den Handelsnamen Abil^{®} Quat 3270, 3272 und 3474 vertrieben. Erfindungsgemäß bevorzugte kosmetische Mittel sind dadurch gekennzeichnet, dass sie, bezogen auf ihr Gewicht, als Polymer b1) 0,05 bis 5,0 Gew.-%, bevorzugt 0,1 bis 3,0 Gew.-%, besonders bevorzugt 0,2 bis 2,0 Gew.-% und insbesondere 0,5 bis 1,5 Gew.-% diquartäres Silikon enthalten.

Zur Gruppe der bevorzugten quartären Polydimethylsiloxane zählen weiterhin die triquartäre Silikone, vorzugsweise triquartäre Silikone der allgemeinen Formel

R¹Si[(OSiMe₂)ₙ-(CH₂)₃-O-CH₂-CH(OH)-CH₂-N⁺Me₂R²]₃ ,

wobei
a) R¹ für Ph
   R² vorzugsweise für -(CH₂)₃NHC(O)C₈-₂₂Alkyl
   n vorzugsweise für eine Zahl von 10 bis 100 steht; oder
b) R¹ für Me
   R² vorzugsweise für -(CH₂)₃NHC(O)C₈₋₂₂Alkyl
   n vorzugsweise für eine Zahl von 10 bis 100 steht.

Ein bevorzugte triquartäres Silikon b1) ist das bereits genannte Silicone Quaternium-22, das beispielsweise von der Firma Evonik unter der Handelsbezeichnung Abil^{®} T-Quat 60 vertrieben wird. Erfindungsgemäß bevorzugte kosmetische Mittel sind dadurch gekennzeichnet, dass sie, bezogen auf ihr Gewicht, als Polymer b1) 0,05 bis 5,0 Gew.-%, bevorzugt 0,1 bis 3,0 Gew.-%, besonders bevorzugt 0,2 bis 2,0 Gew.-% und insbesondere 0,5 bis 1,5 Gew.-% triquartäres Silikon enthalten.

Ein weiteres erfindungsgemäßes Silikon b1) sind Polyammonium-Polysiloxan Verbindungen, wie sie beispielsweise unter der Handelsbezeichnung Baysilone^{®} TP 3911, SME 253 und SFE 839 von GE Bayer Silicones bezogen werden können.

Als Polymer b) sind in den erfindungsgemäßen kosmetischen Mitteln weiterhin die Silsesquioxane b2), insbesondere die Polyalkylsilsesquioxane einsetzbar. Kosmetisches Mittel, bei denen das Silikonpolymer b2) aus der Gruppe der Polyalkylsilsesquioxane ausgewählt ist, werden bevorzugt. Polyalkylsilsesquioxane sind Verbindungen aus der Gruppe der polyedrischen oligomeren Silsesquioxane (POSS), welche durch die empirische Formel RSiO_{1,5} beschrieben werden, wobei R ein organischer Substituent ist, wie zum Beispiel Wasserstoff, Siloxy- oder cyclische oder lineare aliphatische oder aromatische Gruppe, welche zusätzlich reaktive funktionelle Gruppen enthalten kann, zum Beispiel Alkohol-, Ester-, Amin-, Keto-, Olefin-, Ether-oder Halogenidgruppen. Die Basisstruktur von POSS-Verbindungen weist ein polyedrisches Si-O-Rueckgrat auf, an welches die R-Gruppen gebunden sind. Homoleptische POSS-Verbindungen enthaltend lediglich eine einzige Art von R-Gruppen, und heteroleptische POSS-Chemikalien, enthaltend jeweils unterschiedlich R-Gruppen, sind bekannt. Erfindungsgemäß einsetzbar sind beispielsweise die folgenden Polyalkylsilsesquioxane: Isooctyl-POSS[Me₃CCH₂CH(Me)CH₂]ₙTₙ, wobei n = 8, 10 oder 12 ist, Octacyclohexyl-POSS C₄₈H₈₈O₁₂S₁₈, Octacyclopentyl-POSS C₄₀H₇₂O₁₂S₁₈, Octaisobutyl-POSS C₃₂H₇₂O₁₂S₁₈, Octamethyl-POSS C₈H₂₄O₁₂S₁₈. Ganz besonders bevorzugt ist der Einsatz von Polypropylsilsesquioxan als Silikonpolymer b2). In diesen Polypropylsilsesquioxanen ist der Rest R in der Formel RSiO_{1,5} ein Propylrest, wobei unter "Propylrest" sowohl n-Propyl- als auch Isopropylreste verstanden werden.

Der Gewichtsanteil des Silsesquioxans b2) am Gesamtgewicht des kosmetischen Mittels beträgt vorzugsweise 0,05 bis 3,0 Gew.-%, bevorzugt 0,1 bis 2,0 Gew.-%, besonders bevorzugt 0,15 bis 1,5 Gew.-% und insbesondere 0,2 bis 1,0 Gew.-%.

Hinsichtlich ihrer pflegenden Eigenschaften haben sich erfindungsgemäße kosmetische Mittel als besonders vorteilhaft erwiesen, bei denen das Gewichtsverhältnis von Isoparaffin a) zu Polymer b) 50:1 bis 1:1, vorzugsweise 40:1 bis 2:1, besonders bevorzugt 30:1 bis 4:1 und insbesondere 20:1 bis 8:1 beträgt.

Die Zusammensetzung einiger bevorzugter kosmetischer Mittel enthaltend den Inhaltsstoff b) kann den folgenden Tabellen entnommen werden (Angaben in Gew.-% bezogen auf das Gesamtgewicht des kosmetischen Mittels sofern nicht anders angegeben).

| | Formel 17 | Formel 18 | Formel 19 | Formel 20 | Formel 21 |
|---|---|---|---|---|---|
| Isoparaffin * | 5,0 bis 70 | 8,0 bis 60 | 10 bis 50 | 18 | 48 |
| Polymer ** | 0,05 bis 5,0 | 0,1 bis 3,0 | 0,2 bis 2,0 | 0,4 | 1,0 |
| Misc | add 100 | add 100 | add 100 | add 100 | add 100 |

| | Formel 22 | Formel 23 | Formel 24 | Formel 25 | Formel 26 |
|---|---|---|---|---|---|
| Isoparaffin * | 5,0 bis 70 | 8,0 bis 60 | 10 bis 50 | 18 | 48 |
| Polymer *** | 0,05 bis 5,0 | 0,1 bis 3,0 | 0,2 bis 2,0 | 0,1 | 0,5 |
| Misc | add 100 | add 100 | add 100 | add 100 | add 100 |

| | | | | | |
|---|---|---|---|---|---|
| * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. ** R¹Si[(OSiMe₂)ₙ-(CH₂)₃-O-CH₂-CH(OH)-CH₂-N⁺Me₂R²]₃, wobei R¹ für Ph oder Me, R² vorzugsweise für -(CH₂)₃NHC(O)C₈₋₂₂ Alkyl und n vorzugsweise für eine Zahl von 10 bis 100 steht; *** Polypropylsilsesquioxan | | | | | |

Die Zusammensetzung einiger bevorzugter kosmetischer Mittel enthaltend ausgewählte Inhaltsstoff b) kann den folgenden Tabellen entnommen werden (Angaben in Gew.-% bezogen auf das Gesamtgewicht des kosmetischen Mittels sofern nicht anders angegeben).

| | Formel 27 | Formel 28 | Formel 29 | Formel 30 | Formel 31 |
|---|---|---|---|---|---|
| Isoparaffin * | 8,0 bis 60 | 8,0 bis 60 | 10 bis 50 | 18 | 48 |
| Polymer ** | 0,05 bis 5,0 | 0,1 bis 3,0 | 0,2 bis 2,0 | 0,4 | 1,0 |
| Silikonöl | 30 bis 90 | 35 bis 88 | 40 bis 85 | 80 | 50 |
| Misc | add 100 | add 100 | add 100 | add 100 | add 100 |

| | Formel 32 | Formel 33 | Formel 34 | Formel 35 | Formel 36 |
|---|---|---|---|---|---|
| Isoparaffin * | 8,0 bis 60 | 8,0 bis 60 | 10 bis 50 | 18 | 48 |
| Polymer *** | 0,05 bis 5,0 | 0,1 bis 3,0 | 0,2 bis 2,0 | 0,1 | 0,5 |
| Silikonöl **** | 30 bis 90 | 35 bis 88 | 40 bis 85 | 80 | 50 |
| Misc | add 100 | add 100 | add 100 | add 100 | add 100 |

| | Formel 37 | Formel 38 | Formel 39 | Formel 40 | Formel 41 |
|---|---|---|---|---|---|
| Isoparaffin * | 8,0 bis 60 | 8,0 bis 60 | 10 bis 50 | 18 | 48 |
| Polymer ** | 0,05 bis 5,0 | 0,1 bis 3,0 | 0,2 bis 2,0 | 0,4 | 1,0 |
| Silikonöl | 30 bis 90 | 35 bis 88 | 40 bis 85 | 80 | 50 |
| Natürliches Öl | 0,1 bis 10 | 0,2 bis 8,0 | 0,4 bis 5,0 | 0,2 | 1,0 |
| Misc | add 100 | add 100 | add 100 | add 100 | add 100 |

| | Formel 42 | Formel 43 | Formel 44 | Formel 45 | Formel 46 |
|---|---|---|---|---|---|
| Isoparaffin * | 8,0 bis 60 | 8,0 bis 60 | 10 bis 50 | 18 | 48 |
| Polymer *** | 0,05 bis 5,0 | 0,1 bis 3,0 | 0,2 bis 2,0 | 0,1 | 0,5 |
| Silikonöl **** | 30 bis 90 | 35 bis 88 | 40 bis 85 | 80 | 50 |
| Natürliches Öl | 0,1 bis 10 | 0,2 bis 8,0 | 0,4 bis 5,0 | 0,2 | 1,0 |
| Misc | add 100 | add 100 | add 100 | add 100 | add 100 |

| | Formel 47 | Formel 48 | Formel 49 | Formel 50 | Formel 51 |
|---|---|---|---|---|---|
| Isoparaffin * | 8,0 bis 60 | 8,0 bis 60 | 10 bis 50 | 18 | 48 |
| Polymer ** | 0,05 bis 5,0 | 0,1 bis 3,0 | 0,2 bis 2,0 | 0,4 | 1,0 |
| Silikonöl | 30 bis 90 | 35 bis 88 | 40 bis 85 | 60 | 50 |
| Esteröl | 0,1 bis 30 | 0,2 bis 27 | 0,4 bis 24 | 20 | 1,0 |
| Misc | add 100 | add 100 | add 100 | add 100 | add 100 |

| | Formel 52 | Formel 53 | Formel 54 | Formel 55 | Formel 56 |
|---|---|---|---|---|---|
| Isoparaffin * | 8,0 bis 60 | 8,0 bis 60 | 10 bis 50 | 18 | 48 |
| Polymer *** | 0,05 bis 5,0 | 0,1 bis 3,0 | 0,2 bis 2,0 | 0,1 | 0,5 |
| Silikonöl **** | 30 bis 90 | 35 bis 88 | 40 bis 85 | 60 | 50 |
| Esteröl | 0,1 bis 10 | 0,2 bis 8,0 | 0,4 bis 5,0 | 20 | 1,0 |
| Misc | add 100 | add 100 | add 100 | add 100 | add 100 |

| | | | | | |
|---|---|---|---|---|---|
| * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. ** R¹Si[(OSiMe₂)ₙ-(CH₂)₃-O-CH₂-CH(OH)-CH₂-N⁺Me₂R²]₃, wobei R¹ für Ph oder Me, R² vorzugsweise für -(CH₂)₃NHC(O)C₈₋₂₂ Alkyl und n vorzugsweise für eine Zahl von 10 bis 100 steht; *** Polypropylsilsesquioxan **** Mischung aus Dimethiconol und Cyclodimethicon im Gewichtsverhältnis von Dimethiconol zu Cyclodimethicon 1:2 bis 1:10 | | | | | |

Der Bestandteil c) ist eine polymere quartäre Ammoniumverbindung. Die Gruppe der polymeren quartären Ammoniumverbindungen umfasst dabei erfindungsgemäß insbesondere die kationischen Polymere. Diese Polymere c) weisen erfindungsgemäß ein quartäres, also permanent kationisches Stickstoffatom mit vier Substituenten auf. Bei den polymeren quartären Ammoniumverbindungen c) handelt es sich mit anderen Worten um Polymere mit einer "permant" kationischen Ammoniumverbindung. Als "permanent kationisch" werden erfindungsgemäß solche Polymere bezeichnet, die unabhängig vom pH-Wert des Mittels eine kationische Gruppe aufweisen.

Die Zusammensetzung einiger bevorzugter kosmetischer Mittel enthaltend den Inhaltsstoff c) kann den folgenden Tabellen entnommen werden (Angaben in Gew.-% bezogen auf das Gesamtgewicht des kosmetischen Mittels sofern nicht anders angegeben).

| | Formel 57 | Formel 58 | Formel 59 | Formel 60 |
|---|---|---|---|---|
| Isoparaffin * | 0,1 bis 9,0 | 0,2 bis 8,0 | 0,5 bis 6,0 | 1,0 bis 4,0 |
| Polymer **, Misc | add 100 | add 100 | add 100 | add 100 |

| | | | | |
|---|---|---|---|---|
| * Isoparaffingemisch, umfassend mindestens ein Isoparaffin aus der Gruppe Isoundecan, Isododecan und Isotridecan, wobei der Gewichtsanteil des Isoundecans, Isodedecans und Isotridecans am Gesamtgewicht aller in dem kosmetischen Mittel eingesetzten Isoparaffine vorzugsweise mehr als 62 Gew.-%, bevorzugt mehr als 72 Gew.-%, besonders bevorzugt mehr als 82 Gew.-% und insbesondere mehr als 92 Gew.-% beträgt. ** polymere quartäre Ammoniumverbindung | | | | |

| | Formel 61 | Formel 62 | Formel 63 | Formel 64 |
|---|---|---|---|---|
| Isoparaffin * | 0,1 bis 9,0 | 0,2 bis 8,0 | 0,5 bis 6,0 | 1,0 bis 4,0 |
| Polymer **, Misc | add 100 | add 100 | add 100 | add 100 |

| | | | | |
|---|---|---|---|---|
| * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. ** polymere quartäre Ammoniumverbindung | | | | |

Bevorzugte kationische Gruppen sind quartäre Ammoniumgruppen, die über eine C1-4-Kohlenwasserstoffgruppe an eine aus Acrylsäure, Methacrylsäure oder deren Derivaten aufgebaute Polymerhauptkette gebunden sind.

Erfindungsgemäß bevorzugte Polymere zeichnen sich durch eine Ladungsdichte von mindestens 1 bis 7 meq/g aus. Eine Ladungsdichte von mindestens 2 bis 7 meq/g ist dabei bevorzugt. Besonders bevorzugt ist eine Ladungsdichte von mindestens gleich 3meq/g bis 7 meq/g. Bevorzugt sind solche Polymere, die eine ausreichende Löslichkeit in Wasser oder Alkohol besitzen, um in dem erfindungsgemäßen Mittel bei der vollständig in Lösung zu gehen.

Die kationischen Polymere können Homo- oder Copolymere sein, wobei die quartären Stickstoffgruppen entweder in der Polymerkette oder vorzugsweise als Substituent an einem oder mehreren der Monomeren enthalten sind. Geeignete kationische Monomere sind ungesättigte, radikalisch polymerisierbare Verbindungen, welche mindestens eine kationische, quartäre Ammoniumgruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1-bis C7-Alkylgruppen, besonders bevorzugt C1- bis C3-Alkylgruppen sind.

Geeignete Polymere mit quartären Ammoniumgruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium- 11).

Weitere Beispiele für polymere quartäre Ammoniumverbindungen b) sind
- Copolymere des Vinylpyrrolidons mit quaternierten Derivaten des Dialkylaminoalkylacrylats und -methacrylats, beispielsweise Vinylpyrrolidon/Dimethylaminoethylmethacrylatmethosulfat Copolymere, wie sie unter den Handelsbezeichnungen Gafquat^{®} 755 N und Gafquat^{®} 734 von der Firma Gaf Co., USA vertrieben werden;
- Polyvinylpyrrolidon/Imidazoliminmethochlorid Copolymere, wie sie von der Firma BASF, Deutschland unter dem Handelsnamen Luviquat^{®} HM 550 vertrieben werden;
- Terpolymere aus Dimethyldiallylammoniumchlorid, Natriumacrylat und Acrylamid, das von der Firma Calgon/USA unter dem Handelsnamen Merquat^{®} Plus 3300 vertrieben wird;
- Vinylpyrrolidon/Methacrylamidopropyltrimethylammoniumchlorid Copolymer, das von der Firma ISP unter dem Handelsnamen Gafquat^{®} HS 100 vertrieben wird;
- polymere Dimethyldiallylammoniumsalze und deren Copolymere mit Estern und Amiden von Acrylsäure und Methacrylsäure, wie sie unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlich sind;
- Vinylpyrrolidon-Vinylimidazoliummethochlorid-Copolymere, wie sie unter den Bezeichnungen Luviquat^{®} FC 370, FC 550, FC 905 und HM 552 angeboten werden;
- die unter den Bezeichnungen Polyquaternium-24 (Handelsprodukt z. B. Quatrisoft^{®} LM 200), bekannten Polymere; sowie
- die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Besonders bevorzugte kosmetische Mittel sind dadurch gekennzeichnet, dass mindestens eine polymere quartäre Ammoniumverbindung b) ausgewählt ist aus der Gruppe der Homo- oder Copolymere des Methacryloyloxyethyltrimethylammoniumchlorids.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (3V Sigma) im Handel erhältlich.

Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Dieses Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Bevorzugte Copolymere des Methacryloyloxyethyltrimethylammoniumchlorids enthalten als nichtionogene Monomereinheiten Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwässrige Polymerdispersion unter der Bezeichnung Salcare^{®} SC 92 erhältlich. Der Gewichtsanteil der polymeren quartären Ammoniumverbindung b) am Gesamtgewicht des kosmetischen Mittels beträgt vorzugsweise 0,1 bis 10 Gew.-%, bevorzugt 0,2 bis 8,0 Gew.-% und insbesondere 0,5 bis 5,0 Gew.-%.

Hinsichtlich ihrer pflegenden Eigenschaften haben sich erfindungsgemäße kosmetische Mittel als besonders vorteilhaft erwiesen, bei denen das Gewichtsverhältnis von Isoparaffin a) zu polymerer quartärer Ammoniumverbindung c) zwischen 8:1 und 1:8, vorzugsweise zwischen 6:1 und 1:6 und insbesondere zwischen 4:1 und 1:4 beträgt.

Die Zusammensetzung einiger bevorzugter kosmetischer Mittel mit ausgewählten Inhaltsstoffen c) kann den folgenden Tabellen entnommen werden (Angaben in Gew.-% bezogen auf das Gesamtgewicht des kosmetischen Mittels sofern nicht anders angegeben).

| | Formel 65 | Formel 66 | Formel 67 | Formel 68 | Formel 69 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Misc | add 100 | add 100 | add 100 | add 100 | add 100 |

| | | | | | |
|---|---|---|---|---|---|
| * Isoparaffingemisch, umfassend mindestens ein Isoparaffin aus der Gruppe Isoundecan, Isododecan und Isotridecan, wobei der Gewichtsanteil des Isoundecans, Isodedecans und Isotridecans am Gesamtgewicht aller in dem kosmetischen Mittel eingesetzten Isoparaffine vorzugsweise mehr als 62 Gew.-%, bevorzugt mehr als 72 Gew.-%, besonders bevorzugt mehr als 82 Gew.-% und insbesondere mehr als 92 Gew.-% beträgt. ** polymere quartäre Ammoniumverbindung | | | | | |

| | Formel 70 | Formel 71 | Formel 72 | Formel 73 | Formel 74 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Misc | add 100 | add 100 | add 100 | add 100 | add 100 |

| | | | | | |
|---|---|---|---|---|---|
| * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. ** Poly(methacryloyloxyethyltrimethylammoniumchlorid) oder Acrylamid-Methacryloyloxyethyltrimethylammoniumchlorid-Copolymer | | | | | |

Als weiteren wesentlichen Bestandteil enthalten die erfindungsgemäßen kosmetischen Mittel einen Ölkörper d).

Die Zusammensetzung einiger bevorzugter kosmetischer Mittel enthaltend den Inhaltsstoff d) kann den folgenden Tabellen entnommen werden (Angaben in Gew.-% bezogen auf das Gesamtgewicht des kosmetischen Mittels sofern nicht anders angegeben).

| | Formel 75 | Formel 76 | Formel 77 | Formel 78 |
|---|---|---|---|---|
| Isoparaffin * | 10 bis 70 | 12 bis 65 | 15 bis 60 | 50 |
| Ölkörper, Misc | add 100 | add 100 | add 100 | add 100 |

| | | | | |
|---|---|---|---|---|
| * Isoparaffingemisch, umfassend mindestens ein Isoparaffin aus der Gruppe Isoundecan, Isododecan und Isotridecan, wobei der Gewichtsanteil des Isoundecans, Isodedecans und Isotridecans am Gesamtgewicht aller in dem kosmetischen Mittel eingesetzten Isoparaffine vorzugsweise mehr als 62 Gew.-%, bevorzugt mehr als 72 Gew.-%, besonders bevorzugt mehr als 82 Gew.-% und insbesondere mehr als 92 Gew.-% beträgt. | | | | |

| | Formel 79 | Formel 80 | Formel 81 | Formel 82 |
|---|---|---|---|---|
| Isoparaffin * | 10 bis 70 | 12 bis 65 | 15 bis 60 | 50 |
| Ölkörper, Misc | add 100 | add 100 | add 100 | add 100 |

| | | | | |
|---|---|---|---|---|
| * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. | | | | |

Zu den bevorzugten Ölkörpern d) zählen insbesondere:
- Die Silikonöle. Die Silikonöle sind erfindungsgemäß unterschiedlich zu den erfindungsgemäßen Silikonpolymeren der Gruppe b). Zur Gruppe der Silikonöle zählen insbesondere die Dimethicone, zu welchen auch die Cyclomethicone zu rechnen sind, die aminofunktionellen Silikone sowie die Dimethiconole.

Die erfindungsgemäßen Dimethicone können sowohl linear als auch verzweigt als auch cyclisch oder cyclisch und verzweigt sein.

Lineare Dimethicone können durch die folgende Strukturformel (Si1) dargestellt werden:

(SiR¹₃) - O - (SiR²₂ - O - )ₓ - (SiR¹₃) (Si1)

Verzweigte Dimethicone können durch die Strukturformel (Si1.1) dargestellt werden:

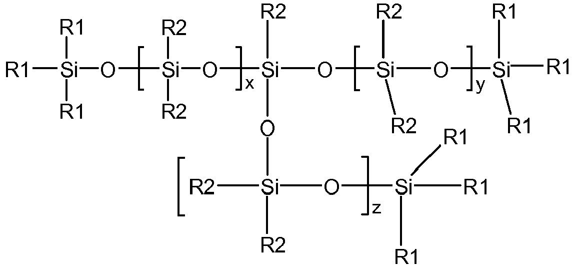

Bevorzugt als R¹ und R² sind Methyl, Phenyl und C2 bis C22 - Alkylreste, insbesondere jedoch Methyl. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen vorzugsweise zwischen 1000 D und 10000000 D. Die Viskositäten liegen vorzugsweise zwischen 100 und 10000000 cPs gemessen bei 25 °C mit Hilfe eines Glaskapillarviskosimeters nach der Dow Corning Corporate Testmethode CTM 0004 vom 20. Juli 1970. Besonders bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Auch die nach INCI als Cyclomethicone bezeichneten cyclischen Dimethicone sind erfindungsgemäß mit Vorzug einsetzbar. Hier sind erfindungsgemäße kosmetische oder dermatologische Zubereitungen bevorzugt, die mindestens ein Silikon der Formel (Si-4) 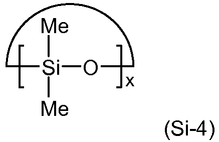 enthalten, in der x für eine Zahl von 3 bis 200, vorzugsweise von 3 bis 10, weiter bevorzugt von 3 bis 7 und insbesondere 3, 4, 5 oder 6, steht.

Dimethiconole (Si8) bilden eine weitere Gruppe der Silikone, welche erfindungsgemäß besonders bevorzugt sind. Die erfindungsgemäßen Dimethiconole können sowohl linear als auch verzweigt als auch cyclisch oder cyclisch und verzweigt sein. Lineare Dimethiconole können durch die folgende Strukturformel (Si8 - I) dargestellt werden:

(SiOHR¹₂) - O - (SiR²₂ - O - )ₓ - (SiOHR¹₂) (Si8 - I)

Verzweigte Dimethiconole können durch die Strukturformel (Si8 - II) dargestellt werden:

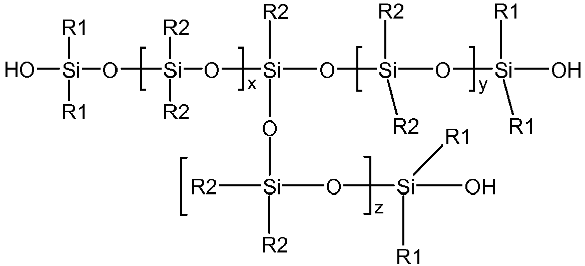

Bevorzugt als R¹ und R² sind Methyl, Phenyl und C2 bis C22 - Alkylreste, insbesondere Methyl. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethiconole liegen vorzugsweise zwischen 1000 D und 10000000 D. Die Viskositäten liegen vorzugsweise zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS ( beide Guardian Laboratories), Nonychosine E, Nonychosine V (beide Exsymol), SanSurf Petrolatum-25, Satin Finish ( beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Besonders bevorzugte erfindungsgemäße Mittel enthalten ein oder mehrere aminofunktionelle Silikone. Solche Silikone können z.B. durch die Formel (Si-2)

M(RₐQ_{b}SiO_{(4-a-b)/2})ₓR_{c}SiO_{(4-c)/2})_{y}M (Si-2)

Beschrieben werden, wobei in der obigen Formel
- R: ein Kohlenwasserstoff oder ein Kohlenwasserstoffrest mit 1 bis etwa 6 Kohlenstoffatomen, vorzugsweise Methyl ist,
- Q: ein polarer Rest der allgemeinen Formel -R¹HZ, vorzugsweise ein Rest der Formel - CH₂CH₂CH₂NHCH₂CH₂NH₂ ist, worin
- R¹: eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und
- Z: ein organischer, aminofunktioneller Rest ist, der mindestens eine aminofunktionelle Gruppe enthält, wobei es sich vorzugsweise um einen -NHCH₂CH ₂NH₂-Rest oder einen Rest der Formel -CH₂CH₂CH₂NHCH₂CH₂NH ₂ handelt;
- a: Werte im Bereich von etwa 0 bis etwa 2 annimmt,
- b: Werte im Bereich von etwa 1 bis etwa 3 annimmt,
- a + b: kleiner als oder gleich 3 ist, und
- c: eine Zahl im Bereich von etwa 1 bis etwa 3 ist, und
- x: eine Zahl im Bereich von 1 bis etwa 2.000, vorzugsweise von etwa 3 bis etwa 50 und am bevorzugtesten von etwa 3 bis etwa 25 ist, und
- y: eine Zahl im Bereich von etwa 20 bis etwa 10.000, vorzugsweise von etwa 125 bis etwa 10.000 und am bevorzugtesten von etwa 150 bis etwa 1.000 ist, und
- M: eine geeignete Silikon-Endgruppe ist, wie sie im Stande der Technik bekannt ist, vorzugsweise Trimethylsiloxy.

Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH ₂-, -CH₂CH₂OCH₂-, -OCH₂CH₂-, - OCH₂ CH₂CH₂-, _CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂₎₃C(O)SCH₂CH₂- ein.

Bevorzugte erfindungsgemäße kosmetische oder dermatologische Zubereitungen enthalten ein aminofunktionelles Silikon der Formel (Si-3)

R'ₐG₃₋ₐ-Si(OSiG ₂)ₙ-(OSiG _{b}R'_{2- b})ₘ-O-SiG₃₋ₐ-R'ₐ (Si-3),

worin bedeutet:
- G: ist -H, eine Phenylgruppe, -OH, -O-CH₃, -CH₃, -O-CH₂CH₃, -CH₂CH₃, -O-CH₂CH₂CH₃, -CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃ ;
- a: steht für eine Zahl zwischen 0 und 3, insbesondere 0;
- b: steht für eine Zahl zwischen 0 und 1, insbesondere 1,
- m und n: sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R': ist ein monovalenter Rest ausgewählt aus
-Q-N(R")-CH₂-CH₂-N(R")₂
-Q-N(R")₂
-Q-N⁺(R")₃A⁻
-Q-N⁺H(R")₂ A⁻
-Q-N'H₂(R")A⁻
-Q-N(R")-CH₂-CH₂-N⁺R"H₂A⁻,
wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Erfindungsgemäß geeignet sind kationische Silikonöle wie beispielsweise die im Handel erhältliche Dow Corning 929 Emulsion (enthaltend ein hydroxylamino-modifiziertes Silikon, das als Amodimethicone bezeichnet wird), Dow Corning 939, Dow Corning 949, Dow Corning 959, DC 2-2078 (Hersteller Dow Corning, INCI-Bezeichnung: Aminopropyl Phenyl Trimethicone), DC 5-7113 (Hersteller Dow Corning, INCI-Bezeichnung: Silicone Quaternium 16), SM-2059 (Hersteller: General Electric) sowie SLM-55067 (Hersteller: Wacker).

Besonders bevorzugte erfindungsgemäße Mittel sind dadurch gekennzeichnet, dass sie mindestens es ein aminofunktionelles Silikon der Formel (Si3-a) 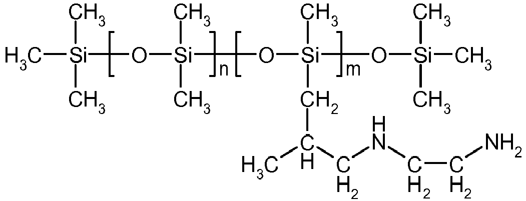 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet und sind beispielsweise unter der Bezeichnung Q2-7224 (Hersteller: Dow Corning; ein stabilisiertes Trimethylsilylamodimethicon) erhältlich.

Besonders bevorzugt sind auch erfindungsgemäße Mittel, die mindestens ein aminofunktionelles Silikon der Formel (Si-3b) 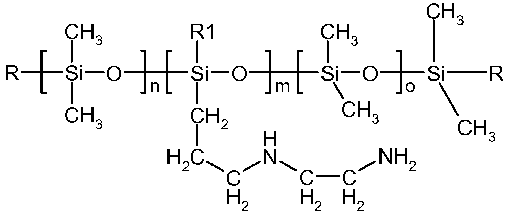 enthalten, worin
R für -OH, eine (gegebenenfalls ethoxylierte und/oder propoxylierte) (C₁ bis C₂₀)-Alkoxygruppe oder eine -CH₃-Gruppe steht,
R' für -OH, eine (C₁ bis C₂₀)-Alkoxygruppe oder eine -CH₃-Gruppe und
m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), bezeichnet.

Ein besonders bevorzugtes funktionalisiertes Aminosilikon entspricht der folgenden Formel: 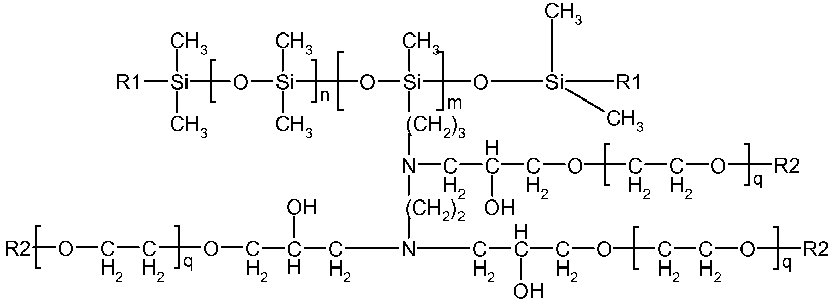
in welcher R1 steht für eine Methyl-, Ethyl-, Hydroxy-, Methoxy- oder Ethoxygruppe,
R2, steht für einen geradkettigen oder verzweigten C8 bis C24 Alkyl- oder Alkylenrest, vorzugsweise einen geradkettigen oder verzweigten C9 bis C22 Alkyl- oder Alkenylrest, besonders bevorzugt einen geradkettigen oder verzweigten C11 bis C18 Alkyl- oder Alkenylrest, höchst bevorzugt einen entsprechenden Alkylrest,
n und m jeweils für ganze Zahlen von 1 bis 1000 stehen und q steht jeweils für eine ganze Zahl von 2 bis 50, bevorzugt 4 bis 30, besonders bevorzugt 4 bis 18 und höchst bevorzugt von 4 bis 12.

Das Molekulargewicht derartiger Verbindungen beträgt 15.000 bis 2.000.000, gemessen mit einem Brookfield Rotationsviskosimeter RV, Spindel 5, bei 20 °C. Bevorzugt beträgt das Molgewicht 30.000 bis 1.750.000 und besonders bevorzugt 50.000 bis 1.500.000. Der Stickstoffgehalt der erfindungsgemäßen Silikone beträgt 0,03 bis 4,2 Gew.%, bevorzugt 0,1 bis 2,8 Gew.% und höchst bevorzugt 0,16 bis 1,4 Gew.%. Erfindungsgemäße aminofunktionelle kationische Silikone der obigen Formel können beispielsweise von der Fa. Clariant bezogen werden. Ein erfindungsgemäß höchst bevorzugtes Produkt ist unter der INCI-Bezeichnung Trideceth-9-Amodimethicone and Trideceth-12 im Handel erhältlich.

Eine erfindungsgemäß höchst bevorzugte Gruppe von aminofunktionalen Silikonen wird im Folgenden beschrieben. Ganz besonders bevorzugt wird daher als aminofunktionales Silikon mindestens ein 4-morpholinomethyl-substituiertes Silikon der Formel (V) verwendet, 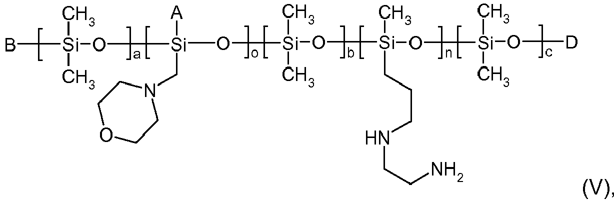 in der
- A: für eine über ein -O- gebundene Struktureinheit (I), (II) oder (III) 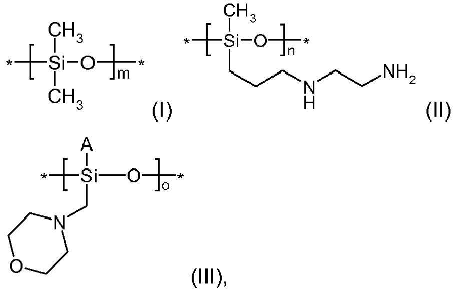 oder einen über ein -O- gebundenen oligomeren oder polymeren Rest enthaltend Struktureinheiten der Formeln (I), (II) oder (III) oder die Hälfte eines verbindenden O-Atoms zu einer Struktureinheit (III) oder für -OH steht,
- *: für eine Bindung zu einer der Struktureinheiten (I), (II) oder (III) oder für eine Endgruppe B (Si-gebunden) oder D (O-gebunden) steht,
- B: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
- a, b und c: für ganze Zahlen zwischen 0 und 1000 stehen, mit der Maßgabe a + b + c > 0
- m, n und o: für ganze Zahlen zwischen 1 und 1000 stehen.

Derartige aminofunktionale Silikone tragen die INCI - Bezeichnung Amodimethicone/Morpholinomethyl Silsesquioxane Copolymer. Ein besonders geeignetes Amodimethicone ist das Produkt mit der Handelsbezeichnung Wacker Belsil® ADM 8301 E.

Die aminofunktionellen und wie zuvor beschriebenen Silikonpolymere sind in den erfindungsgemäßen Zusammensetzungen in Mengen von 0,01 bis 5 Gew.%, bevorzugt in Mengen von 0,05 bis 5 Gew.% und ganz besonders bevorzugt in Mengen von 0,1 bis 5 Gew.% enthalten. Die allerbesten Ergebnisse werden dabei mit Mengen von 0,1 bis 2,5 Gew.% jeweils bezogen auf die Gesamtzusammensetzung des jeweiligen Mittels erhalten.

Unabhängig davon, welche aminofunktionellen Silikone eingesetzt werden, sind erfindungsgemäße kosmetische Zubereitungen bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silikons. Sie kann durch Titration ermittelt und auch in der Einheit mg KOH/g angegeben werden.

Erfindungsgemäß bevorzugt ist die Verwendung einer Mischung von Silikonölen. Diese Mischung von Silikonölen enthält vorzugsweise mindestens ein Dimethiconol. Besonders bevorzugt ist die Kombination eines Dimethiconols mit einem Dimethicon, vorzugsweise einem Cyclodimethicon. In bevorzugten Mischungen aus Dimethiconol und Cyclodimethicon beträgt das Gewichtsverhältnis von Dimethiconol zu Cyclodimethicon 1:2 bis 1:10, vorzugsweise 1:3 bis 1:9 und insbesondere 1:4 bis 1:8.

Bevorzugte erfindungsgemäße kosmetische Mittel sind dadurch gekennzeichnet, dass es sich bei dem Ölkörper d) um ein Silikonöl, vorzugsweise um ein Dimethiconol handelt. Der Gewichtsanteil des Silikonöls am Gesamtgewicht bevorzugter kosmetischer Mittel beträgt 15 bis 90 Gew.-%, vorzugsweise 20 bis 90 Gew.-%, besonders bevorzugt 25 bis 88 Gew.-% und insbesondere 30 bis 85 Gew.-%. Dies bedeutet, wenn nur ein erfindungsgemäßes Silikon verwendet wird, ist dieses mit mindestens 15 Gew.% bezogen auf die Gesamtzusammensetzung enthalten. Wenn mehrere erfindungsgemäße Silikone verwendet werden, dann muss die Summe der Mengen der einzelnen Silikone mindestens 15 Gew.% betragen. Besonders bevorzugte kosmetische Mittel enthalten bezogen auf ihr Gesamtgewicht 2 bis 20 Gew.-%, vorzugsweise 3 bis 18 Gew.-%, besonders bevorzugt 4 bis 16 Gew.-% und insbesondere 5 bis 14 Gew.-% Dimethiconol.

Weiterhin zählen zu den erfindungsgemäßen Ölkörpern:
- pflanzliche Öle. Beispiele für solche Öle sind Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Haselnussöl, Holundersamenöl, Johannesbeersamenöl, Jojobaöl, Kakaobutter, Leinöl, Macadamianussöl, Maiskeimöl, Mandelöl, Marulaöl, Nachtkerzenöl, Olivenöl, Orangenöl, Palmöl, Pfirsichkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl, Weizenkeimöl, Wildrosenöl und die flüssigen Anteile des Kokosöls. Geeignet sind aber auch andere Triglyceridöle wie die flüssigen Anteile des Rindertalgs sowie synthetische Triglyceridöle. Bevorzugte erfindungsgemäße kosmetische Mittel sind dadurch gekennzeichnet, dass es sich bei dem Ölkörper d) um ein natürliches Öl handelt. Der Gewichtsanteil des natürlichen Öls am Gesamtgewicht bevorzugter kosmetischer Mittel beträgt 0,1 bis 10 Gew.-%, vorzugsweise 0,2 bis 8,0 Gew.% und insbesondere 0,4 bis 5,0 Gew.-%.
- flüssige Paraffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®}OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V). Bevorzugte erfindungsgemäße kosmetische Mittel sind dadurch gekennzeichnet, dass es sich bei dem Ölkörper b) um ein Esteröl, vorzugsweise um Isopropylmyristat handelt. Der Gewichtsanteil des Esteröls am Gesamtgewicht bevorzugter kosmetischer Mittel beträgt 0,1 bis 30 Gew.-%, vorzugsweise 0,2 bis 27 Gew.% und insbesondere 0,4 bis 24 Gew.-%.
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- Fettalkohole. Zur Gruppe der bevorzugten Fettalkohole zählen die C₈ bis C₂₂ Fettalkohole, vorzugsweise die C₁₀ bis C₂₀ Fettalkohole und insbesondere die C₁₂ bis C₁₈ Fettalkohole. Bevorzugte erfindungsgemäße kosmetische Mittel sind dadurch gekennzeichnet, dass es sich bei dem Ölkörper d) um einen Fettalkohol handelt. Der Gewichtsanteil des Fettalkohols am Gesamtgewicht bevorzugter kosmetischer Mittel beträgt 0,1 bis 10 Gew.-%, vorzugsweise 0,2 bis 8,0 Gew.% und insbesondere 0,4 bis 5,0 Gew.-%.
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I), 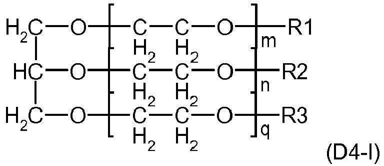 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Der Gewichtsanteil des Ölkörpers am Gesamtgewicht erfindungsgemäß bevorzugter kosmetischer Mittel beträgt 30 bis 90 Gew.-%, vorzugsweise 35 bis 88 Gew.% und insbesondere 40 bis 85 Gew.-%.

Hinsichtlich ihrer pflegenden Eigenschaften haben sich erfindungsgemäße kosmetische Mittel als besonders vorteilhaft erwiesen, bei denen das Gewichtsverhältnis von Isoparaffin a) zu Ölkörper d) 6:1 bis 1:6, vorzugsweise 4:1 bis 1:4 und insbesondere 2:1 bis 1:2 beträgt.

Die Zusammensetzung einiger bevorzugter kosmetischer Mittel enthaltend den Inhaltsstoff d) kann den folgenden Tabellen entnommen werden (Angaben in Gew.-% bezogen auf das Gesamtgewicht des kosmetischen Mittels sofern nicht anders angegeben).

| | Formel 83 | Formel 84 | Formel 85 | Formel 86 | Formel 87 |
|---|---|---|---|---|---|
| Isoparaffin * | 10 bis 70 | 12 bis 65 | 15 bis 60 | 40 | 14 |
| Silikonöl | 20 bis 90 | 25 bis 88 | 30 bis 85 | 35 | 80 |
| Misc | add 100 | add 100 | add 100 | add 100 | add 100 |

| | Formel 88 | Formel 89 | Formel 90 | Formel 91 | Formel 92 |
|---|---|---|---|---|---|
| Isoparaffin * | 10 bis 70 | 12 bis 65 | 15 bis 60 | 40 | 14 |
| Silikonöl ** | 20 bis 90 | 25 bis 88 | 30 bis 85 | 35 | 80 |
| Misc | add 100 | add 100 | add 100 | add 100 | add 100 |

| | Formel 93 | Formel 94 | Formel 95 | Formel 96 | Formel 97 |
|---|---|---|---|---|---|
| Isoparaffin * | 10 bis 70 | 12 bis 65 | 15 bis 60 | 40 | 14 |
| natürliches Öl** | 0,1 bis 10 | 0,2 bis 8,0 | 0,4 bis 5,0 | 0,2 | 0,6 |
| Misc | add 100 | add 100 | add 100 | add 100 | add 100 |

| | Formel 98 | Formel 99 | Formel 100 | Formel 101 | Formel 102 |
|---|---|---|---|---|---|
| Isoparaffin * | 10 bis 70 | 10 bis 70 | 12 bis 65 | 40 | 14 |
| Silikonöl | 25 bis 88 | 25 bis 88 | 30 bis 85 | 35 | 80 |
| natürliches Öl | 0,1 bis 10 | 0,2 bis 8,0 | 0,4 bis 5,0 | 0,2 | 0,6 |
| Misc | add 100 | add 100 | add 100 | add 100 | add 100 |

| | Formel 103 | Formel 104 | Formel 105 | Formel 106 | Formel 107 |
|---|---|---|---|---|---|
| Isoparaffin * | 10 bis 70 | 10 bis 70 | 12 bis 65 | 40 | 14 |
| Silikonöl ** | 25 bis 88 | 25 bis 88 | 30 bis 85 | 35 | 80 |
| natürliches Öl | 0,1 bis 10 | 0,2 bis 8,0 | 0,4 bis 5,0 | 0,2 | 0,6 |
| Misc | add 100 | add 100 | add 100 | add 100 | add 100 |

| | Formel 108 | Formel 109 | Formel 110 | Formel 111 | Formel 112 |
|---|---|---|---|---|---|
| Isoparaffin * | 10 bis 70 | 12 bis 65 | 15 bis 60 | 40 | 14 |
| Esteröl | 0,1 bis 30 | 0,2 bis 27 | 0,4 bis 24 | 20 | 0,6 |
| Misc | add 100 | add 100 | add 100 | add 100 | add 100 |

| | Formel 113 | Formel 114 | Formel 115 | Formel 116 | Formel 117 |
|---|---|---|---|---|---|
| Isoparaffin * | 10 bis 70 | 12 bis 65 | 15 bis 60 | 40 | 14 |
| Isopropylmyristat | 0,1 bis 30 | 0,2 bis 27 | 0,4 bis 24 | 20 | 0,6 |
| Misc | add 100 | add 100 | add 100 | add 100 | add 100 |

| | Formel 118 | Formel 119 | Formel 120 | Formel 121 | Formel 122 |
|---|---|---|---|---|---|
| Isoparaffin * | 10 bis 70 | 10 bis 70 | 12 bis 65 | 40 | 14 |
| Silikonöl | 25 bis 88 | 25 bis 88 | 30 bis 85 | 35 | 80 |
| Esteröl | 0,1 bis 30 | 0,2 bis 27 | 0,4 bis 24 | 20 | 0,6 |
| Misc | add 100 | add 100 | add 100 | add 100 | add 100 |

| | Formel 123 | Formel 124 | Formel 125 | Formel 126 | Formel 127 |
|---|---|---|---|---|---|
| Isoparaffin * | 10 bis 70 | 10 bis 70 | 12 bis 65 | 40 | 14 |
| Silikonöl | 25 bis 88 | 25 bis 88 | 30 bis 85 | 35 | 80 |
| Isopropylmyristat | 0,1 bis 30 | 0,2 bis 27 | 0,4 bis 24 | 20 | 0,6 |
| Misc | add 100 | add 100 | add 100 | add 100 | add 100 |

| | Formel 128 | Formel 129 | Formel 130 | Formel 131 | Formel 132 |
|---|---|---|---|---|---|
| Isoparaffin * | 10 bis 70 | 10 bis 70 | 12 bis 65 | 40 | 14 |
| Silikonöl ** | 25 bis 88 | 25 bis 88 | 30 bis 85 | 35 | 80 |
| Esteröl | 0,1 bis 30 | 0,2 bis 27 | 0,4 bis 24 | 20 | 0,6 |
| Misc | add 100 | add 100 | add 100 | add 100 | add 100 |

| | Formel 133 | Formel 134 | Formel 135 | Formel 136 | Formel 137 |
|---|---|---|---|---|---|
| Isoparaffin * | 10 bis 70 | 10 bis 70 | 12 bis 65 | 40 | 14 |
| Silikonöl ** | 25 bis 88 | 25 bis 88 | 30 bis 85 | 35 | 80 |
| Isopropylmyristat | 0,1 bis 30 | 0,2 bis 27 | 0,4 bis 24 | 20 | 0,6 |
| Misc | add 100 | add 100 | add 100 | add 100 | add 100 |

| | Formel 138 | Formel 139 | Formel 140 | Formel 141 | Formel 142 |
|---|---|---|---|---|---|
| Isoparaffin * | 10 bis 70 | 10 bis 70 | 12 bis 65 | 40 | 14 |
| Silikonöl | 25 bis 88 | 25 bis 88 | 30 bis 85 | 35 | 80 |
| Natürliches Öl | 0,1 bis 10 | 0,2 bis 8,0 | 0,4 bis 5,0 | 0,2 | 0,6 |
| Esteröl | 0,1 bis 30 | 0,2 bis 27 | 0,4 bis 24 | 20 | 0,6 |
| Misc | add 100 | add 100 | add 100 | add 100 | add 100 |

| | Formel 143 | Formel 144 | Formel 145 | Formel 146 | Formel 147 |
|---|---|---|---|---|---|
| Isoparaffin * | 10 bis 70 | 10 bis 70 | 12 bis 65 | 40 | 14 |
| Silikonöl | 25 bis 88 | 25 bis 88 | 30 bis 85 | 35 | 80 |
| Natürliches Öl | 0,1 bis 10 | 0,2 bis 8,0 | 0,4 bis 5,0 | 0,2 | 0,6 |
| Isopropylmyristat | 0,1 bis 30 | 0,2 bis 27 | 0,4 bis 24 | 20 | 0,6 |
| Misc | add 100 | add 100 | add 100 | add 100 | add 100 |

| | Formel 148 | Formel 149 | Formel 150 | Formel 151 | Formel 152 |
|---|---|---|---|---|---|
| Isoparaffin * | 10 bis 70 | 10 bis 70 | 12 bis 65 | 40 | 14 |
| Silikonöl ** | 25 bis 88 | 25 bis 88 | 30 bis 85 | 35 | 80 |
| Natürliches Öl | 0,1 bis 10 | 0,2 bis 8,0 | 0,4 bis 5,0 | 0,2 | 0,6 |
| Esteröl | 0,1 bis 30 | 0,2 bis 27 | 0,4 bis 24 | 20 | 0,6 |
| Misc | add 100 | add 100 | add 100 | add 100 | add 100 |

| | Formel 153 | Formel 154 | Formel 155 | Formel 156 | Formel 157 |
|---|---|---|---|---|---|
| Isoparaffin * | 10 bis 70 | 10 bis 70 | 12 bis 65 | 40 | 14 |
| Silikonöl ** | 25 bis 88 | 25 bis 88 | 30 bis 85 | 35 | 80 |
| Natürliches Öl | 0,1 bis 10 | 0,2 bis 8,0 | 0,4 bis 5,0 | 0,2 | 0,6 |
| Isopropylmyristat | 0,1 bis 30 | 0,2 bis 27 | 0,4 bis 24 | 20 | 0,6 |
| Misc | add 100 | add 100 | add 100 | add 100 | add 100 |

| | Formel 158 | Formel 159 | Formel 160 | Formel 161 | Formel 162 |
|---|---|---|---|---|---|
| Isoparaffin * | 10 bis 70 | 10 bis 70 | 12 bis 65 | 40 | 14 |
| Silikonöl | 25 bis 88 | 25 bis 88 | 30 bis 85 | 35 | 80 |
| Macadamianussöl | 0,1 bis 3,0 | 0,1 bis 2,0 | 0,1 bis 1,0 | 0,2 | 0,15 |
| Arganöl | 0,1 bis 3,0 | 0,1 bis 2,0 | 0,1 bis 1,0 | .. | 0,15 |
| Aprikosenkernöl | 0,1 bis 3,0 | 0,1 bis 2,0 | 0,1 bis 1,0 | .. | 0,15 |
| Olivenöl | 0,1 bis 3,0 | 0,1 bis 2,0 | 0,1 bis 1,0 | .. | 0,15 |
| Esteröl | 0,1 bis 30 | 0,2 bis 27 | 0,4 bis 24 | 20 | 0,6 |
| Misc | add 100 | add 100 | add 100 | add 100 | add 100 |

| | Formel 163 | Formel 164 | Formel 165 | Formel 166 | Formel 167 |
|---|---|---|---|---|---|
| Isoparaffin * | 10 bis 70 | 10 bis 70 | 12 bis 65 | 40 | 14 |
| Silikonöl | 25 bis 88 | 25 bis 88 | 30 bis 85 | 35 | 80 |
| Macadamianussöl | 0,1 bis 3,0 | 0,1 bis 2,0 | 0,1 bis 1,0 | 0,2 | 0,15 |
| Arganöl | 0,1 bis 3,0 | 0,1 bis 2,0 | 0,1 bis 1,0 | .. | 0,15 |
| Aprikosenkernöl | 0,1 bis 3,0 | 0,1 bis 2,0 | 0,1 bis 1,0 | .. | 0,15 |
| Olivenöl | 0,1 bis 3,0 | 0,1 bis 2,0 | 0,1 bis 1,0 | .. | 0,15 |
| Isopropylmyristat | 0,1 bis 30 | 0,2 bis 27 | 0,4 bis 24 | 20 | 0,6 |
| Misc | add 100 | add 100 | add 100 | add 100 | add 100 |

| | Formel 168 | Formel 169 | Formel 170 | Formel 171 | Formel 172 |
|---|---|---|---|---|---|
| Isoparaffin * | 10 bis 70 | 10 bis 70 | 12 bis 65 | 40 | 14 |
| Silikonöl ** | 25 bis 88 | 25 bis 88 | 30 bis 85 | 35 | 80 |
| Macadamianussöl | 0,1 bis 3,0 | 0,1 bis 2,0 | 0,1 bis 1,0 | 0,2 | 0,15 |
| Arganöl | 0,1 bis 3,0 | 0,1 bis 2,0 | 0,1 bis 1,0 | .. | 0,15 |
| Aprikosenkernöl | 0,1 bis 3,0 | 0,1 bis 2,0 | 0,1 bis 1,0 | .. | 0,15 |
| Olivenöl | 0,1 bis 3,0 | 0,1 bis 2,0 | 0,1 bis 1,0 | .. | 0,15 |
| Esteröl | 0,1 bis 30 | 0,2 bis 27 | 0,4 bis 24 | 20 | 0,6 |
| Misc | add 100 | add 100 | add 100 | add 100 | add 100 |

| | Formel 173 | Formel 174 | Formel 175 | Formel 176 | Formel 177 |
|---|---|---|---|---|---|
| Isoparaffin * | 10 bis 70 | 10 bis 70 | 12 bis 65 | 40 | 14 |
| Silikonöl ** | 25 bis 88 | 25 bis 88 | 30 bis 85 | 35 | 80 |
| Macadamianussöl | 0,1 bis 3,0 | 0,1 bis 2,0 | 0,1 bis 1,0 | 0,2 | 0,15 |
| Arganöl | 0,1 bis 3,0 | 0,1 bis 2,0 | 0,1 bis 1,0 | | 0,15 |
| Aprikosenkernöl | 0,1 bis 3,0 | 0,1 bis 2,0 | 0,1 bis 1,0 | | 0,15 |
| Olivenöl | 0,1 bis 3,0 | 0,1 bis 2,0 | 0,1 bis 1,0 | | 0,15 |
| Isopropylmyristat | 0,1 bis 30 | 0,2 bis 27 | 0,4 bis 24 | 20 | 0,6 |
| Misc | add 100 | add 100 | add 100 | add 100 | add 100 |

| | | | | | |
|---|---|---|---|---|---|
| * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. ** Mischung aus Dimethiconol und Cyclodimethicon im Gewichtsverhältnis von Dimethiconol zu Cyclodimethicon 1:2 bis 1:10 | | | | | |

Als bevorzugten weiteren Bestandteil enthalten die erfindungsgemäßen kosmetischen Mittel eine monomere quartäre Ammoniumverbindung e). Bevorzugte erfindungsgemäße kosmetische Mittel sind dadurch gekennzeichnet, dass sie als weiteren Bestandteil mindestens eine monomere quartäre Ammoniumverbindung e) enthalten.

Als für die haarpflegenden Eigenschaften der Haarbehandlungsmittel hat es sich als vorteilhaft erwiesen, dass diese, bezogen auf ihr Gesamtgewicht, 0,1 bis 10,0 Gew.-%, vorzugsweise 0,25 bis 8,0 Gew.-%, bevorzugt 0,3 bis 6,0 Gew.-% und insbesondere 0,6 bis 5,0 Gew.-% der monomeren quartären Ammoniumverbindung e) enthalten.

Aus der Vielzahl an möglichen quartären Ammoniumverbindungen haben sich die Verbindungen aus den Gruppen:
- Trimethylalkylammoniumhalogenide;
- der Esterquats
- der quartären Imidazoline

Zur Gruppe der Trimethylalkylammoniumhalogenide zählen insbesondere die Verbindungen der Formel (Tkat1-1).

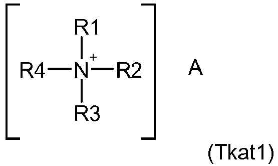

In der Formel (Tkat1) stehen R1, R2, R3 und R4 für jeweils unabhängig voneinander für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann. A steht für ein physiologisch verträgliches Anion, beispielsweise Halogenide wie Chlorid oder Bromid sowie Methosulfate.

Beispiele für Verbindungen der Formel (Tkat1) sind Lauryltrimehtylammoniumchlorid, Cetyltrimethylammoniumchlorid, Cetyltrimethylammoniumbromid, Cetyltrimethylammoniummethosulfat, Dicetyldimethylammoniumchlorid, Tricetylmethylammoniumchlorid, Stearyltrimethylammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat.

Weitere erfindungsgemäß besonders bevorzugte quartären Ammoniumverbindungen sind die kationischen Betainestern der Formel (Tkat1-2.1).

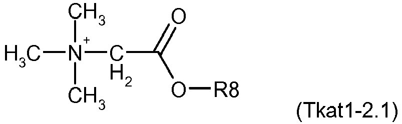

Besonders bevorzugt sind die Esterquats mit den Handelsbezeichnungen Armocare^{®} VGH-70, sowie Dehyquart^{®} F-75, Dehyquart^{®} L80, Stepantex^{®} VS 90 und Akypoquat^{®} 131.

Eine weitere Gruppe sind quartäre Imidazolinverbindungen. Die im Folgenden dargestellte Formel (Tkat2) zeigt die Struktur dieser Verbindungen.

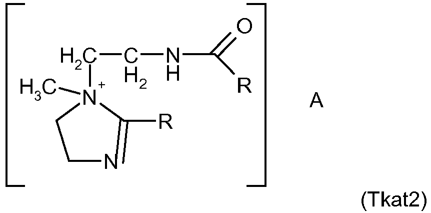

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (Tkat2) enthalten für R jeweils den gleichen Kohlenwasserstoffrest. Die Kettenlänge der Reste R beträgt bevorzugt 12 bis 21 Kohlenstoffatome. A steht für ein Anion wie zuvor beschrieben. Besonders erfindungsgemäße Beispiele sind beispielsweise unter den INCI - Bezeichnungen Quaternium-27, Quaternium-72, Quaternium-83, Quaternium-87 und Quaternium-91 erhältlich. Höchst bevorzugt ist erfindungsgemäß Quaternium-91.

Die Zusammensetzung einiger bevorzugter kosmetischer Mittel enthaltend den Inhaltsstoff e) kann den folgenden Tabellen entnommen werden (Angaben in Gew.-% bezogen auf das Gesamtgewicht des kosmetischen Mittels sofern nicht anders angegeben).

| | Formel 178 | Formel 179 | Formel 180 | Formel 181 | Formel 182 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Trimethylalkylammonium-halogenid | 0,1 bis 10 | 0,25 bis 8,0 | 0,3 bis 6,0 | 0,6 bis 5,0 | 0,8 |
| Misc | add 100 | add 100 | add 100 | add 100 | add 100 |

| | Formel 183 | Formel 184 | Formel 185 | Formel 186 | Formel 187 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| quartäres Imidazolin | 0,1 bis 10 | 0,25 bis 8,0 | 0,3 bis 6,0 | 0,6 bis 5,0 | 0,8 |
| Misc | add 100 | add 100 | add 100 | add 100 | add 100 |

| | | | | | |
|---|---|---|---|---|---|
| * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. ** polymere quartäre Ammoniumverbindung, vorzugsweise Poly(methacryloyloxyethyltrimethylammoniumchlorid) oder Acrylamid-Methacryloyloxyethyltrimethylammoniumchlorid-Copolymer | | | | | |

Neben den zuvor beschriebenen Wirkstoffen können die erfindungsgemäßen kosmetischen Mittel weitere Inhaltsstoffe enthalten. Zur Gruppe dieser weiteren Inhaltsstoffe zählen insbesondere die kosmetisch wirksamen Hilfs- und Zusatzstoffe.

Als geeignete Hilfs- und Zusatzstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin und/oder Propylenglykol erhöht der Zusatz von Panthenol die Flexibilität des bei Anwendung des erfindungsgemäßen Mittels gebildeten Polymerfilms.

Als Pflegestoff können die erfindungsgemäßen Mittel weiterhin mindestens einen Pflanzenextrakt, aber auch Mono- bzw. Oligosaccharide und/oder Lipide enthalten.

In einer Ausführungsform werden die erfindungsgemäßen kosmetischen Mittel vorzugsweise als wässrige Anwendungsmischungen formuliert. Der Wassergehalt bevorzugter kosmetischer Mittel beträgt, bezogen auf ihr Gesamtgewicht, weniger als 10 Gew.-%, vorzugsweise weniger als 5,0 Gew.-%, besonders bevorzugt weniger als 1,0 Gew.-% und insbesondere weniger als 0,1 Gew.-%.

In einer weiteren Ausführungsform werden die erfindungsgemäßen kosmetischen Mittel vorzugsweise als wässrige Anwendungsmischungen formuliert. Der Wassergehalt bevorzugter kosmetischer Mittel beträgt, bezogen auf ihr Gesamtgewicht, 40 bis 95 Gew.-%, vorzugsweise 50 bis 92 Gew.-%, besonders bevorzugt 60 bis 90 Gew.-% und insbesondere 70 bis 88 Gew.-%. Wie eingangs ausgeführt, eignet sich die erfindungsgemäße Wirkstoffkombination aus Isoparaffin und Silikonpolymer b) insbesondere als Trisiloxanersatz und damit für den Einsatz in Trisiloxan freien oder Trisiloxan armen haarkosmetischen Mitten.

Wie eingangs ausgeführt, eignet sich die erfindungsgemäße Wirkstoffkombination aus Isoparaffin und polymerer quartärer Ammoniumverbindung insbesondere als Silikonersatz und damit für den Einsatz in Silikon freien oder Silikon armen haarkosmetischen Mitten.

Bevorzugte erfindungsgemäße Ausführungsformen sind daher dadurch gekennzeichnet, dass das kosmetische Mittel, bezogen auf sein Gesamtgewicht, weniger als 5,0 Gew.-%, vorzugsweise weniger als 2,0 Gew.-%, bevorzugt weniger als 0,5 Gew.-% und insbesondere kein Trisiloxan enthält.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 188 | Formel 189 | Formel 190 | Formel 191 | Formel 192 |
|---|---|---|---|---|---|
| Isoparaffin * | 8,0 bis 60 | 8,0 bis 60 | 10 bis 50 | 18 | 48 |
| Polymer ** | 0,05 bis 5,0 | 0,1 bis 3,0 | 0,2 bis 2,0 | 0,4 | 1,0 |
| Silikonöl | 30 bis 90 | 35 bis 88 | 40 bis 85 | 80 | 50 |
| Parfüm | 0,05 bis 2,0 | 0,1 bis 1,5 | 0,1 bis 1,0 | 0,2 | 0,8 |
| Misc | add 100 | add 100 | add 100 | add 100 | add 100 |

| | Formel 193 | Formel 194 | Formel 195 | Formel 196 | Formel 197 |
|---|---|---|---|---|---|
| Isoparaffin * | 8,0 bis 60 | 8,0 bis 60 | 10 bis 50 | 18 | 48 |
| Polymer *** | 0,05 bis 5,0 | 0,1 bis 3,0 | 0,2 bis 2,0 | 0,1 | 0,5 |
| Silikonöl **** | 30 bis 90 | 35 bis 88 | 40 bis 85 | 80 | 50 |
| Parfüm | 0,05 bis 2,0 | 0,1 bis 1,5 | 0,1 bis 1,0 | 0,2 | 0,8 |
| Misc | add 100 | add 100 | add 100 | add 100 | add 100 |

| | Formel 198 | Formel 199 | Formel 200 | Formel 201 | Formel 202 |
|---|---|---|---|---|---|
| Polymer ** | 0,05 bis 5,0 | 0,1 bis 3,0 | 0,2 bis 2,0 | 0,4 | 1,0 |
| Silikonöl | 30 bis 90 | 35 bis 88 | 40 bis 85 | 80 | 50 |
| Parfüm | 0,05 bis 2,0 | 0,1 bis 1,5 | 0,1 bis 1,0 | 0,2 | 0,8 |
| Isoparaffin * | add 100 | add 100 | add 100 | add 100 | add 100 |

| | Formel 203 | Formel 204 | Formel 205 | Formel 206 | Formel 207 |
|---|---|---|---|---|---|
| Polymer *** | 0,05 bis 5,0 | 0,1 bis 3,0 | 0,2 bis 2,0 | 0,1 | 0,5 |
| Silikonöl **** | 30 bis 90 | 35 bis 88 | 40 bis 85 | 80 | 50 |
| Parfüm | 0,05 bis 2,0 | 0,1 bis 1,5 | 0,1 bis 1,0 | 0,2 | 0,8 |
| Isoparaffin * | add 100 | add 100 | add 100 | add 100 | add 100 |

| | Formel 208 | Formel 209 | Formel 210 | Formel 211 | Formel 212 |
|---|---|---|---|---|---|
| Polymer ** | 0,05 bis 5,0 | 0,1 bis 3,0 | 0,2 bis 2,0 | 0,4 | 1,0 |
| Silikonöl | 30 bis 90 | 35 bis 88 | 40 bis 85 | 80 | 50 |
| Isoparaffin *, evtl. Parfüm | add 100 | add 100 | add 100 | add 100 | add 100 |

| | Formel 213 | Formel 214 | Formel 215 | Formel 216 | Formel 217 |
|---|---|---|---|---|---|
| Polymer *** | 0,05 bis 5,0 | 0,1 bis 3,0 | 0,2 bis 2,0 | 0,1 | 0,5 |
| Silikonöl **** | 30 bis 90 | 35 bis 88 | 40 bis 85 | 80 | 50 |
| Isoparaffin *, evtl. Parfüm | add 100 | add 100 | add 100 | add 100 | add 100 |

| | Formel 218 | Formel 219 | Formel 220 | Formel 221 | Formel 222 |
|---|---|---|---|---|---|
| Polymer ** | 0,05 bis 5,0 | 0,1 bis 3,0 | 0,2 bis 2,0 | 0,4 | 1,0 |
| Silikonöl | 30 bis 90 | 35 bis 88 | 40 bis 85 | 80 | 50 |
| Natürliches Öl | 0,1 bis 10 | 0,2 bis 8,0 | 0,4 bis 5,0 | 0,2 | 1,0 |
| Isoparaffin *, evtl. Parfüm | add 100 | add 100 | add 100 | add 100 | add 100 |

| | Formel 223 | Formel 224 | Formel 225 | Formel 226 | Formel 227 |
|---|---|---|---|---|---|
| Polymer *** | 0,05 bis 5,0 | 0,1 bis 3,0 | 0,2 bis 2,0 | 0,1 | 0,5 |
| Silikonöl **** | 30 bis 90 | 35 bis 88 | 40 bis 85 | 80 | 50 |
| Natürliches Öl | 0,1 bis 10 | 0,2 bis 8,0 | 0,4 bis 5,0 | 0,2 | 1,0 |
| Isoparaffin *, evtl. Parfüm | add 100 | add 100 | add 100 | add 100 | add 100 |

| | Formel 228 | Formel 229 | Formel 230 | Formel 231 | Formel 232 |
|---|---|---|---|---|---|
| Polymer ** | 0,05 bis 5,0 | 0,1 bis 3,0 | 0,2 bis 2,0 | 0,4 | 1,0 |
| Silikonöl | 30 bis 90 | 35 bis 88 | 40 bis 85 | 60 | 50 |
| Esteröl | 0,1 bis 30 | 0,2 bis 27 | 0,4 bis 24 | 20 | 1,0 |
| Isoparaffin *, evtl. Parfüm | add 100 | add 100 | add 100 | add 100 | add 100 |

| | Formel 233 | Formel 234 | Formel 235 | Formel 236 | Formel 237 |
|---|---|---|---|---|---|
| Polymer *** | 0,05 bis 5,0 | 0,1 bis 3,0 | 0,2 bis 2,0 | 0,1 | 0,5 |
| Silikonöl **** | 30 bis 90 | 35 bis 88 | 40 bis 85 | 60 | 50 |
| Esteröl | 0,1 bis 10 | 0,2 bis 8,0 | 0,4 bis 5,0 | 20 | 1,0 |
| Isoparaffin *, evtl. Parfüm | add 100 | add 100 | add 100 | add 100 | add 100 |

| | | | | | |
|---|---|---|---|---|---|
| * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. ** R¹Si[(OSiMe₂)ₙ-(CH₂)₃-O-CH₂-CH(OH)-CH₂-N⁺Me₂R²]₃ , wobei R¹ für Ph oder Me, R² vorzugsweise für -(CH₂)₃NHC(O)C₈₋₂₂ Alkyl und n vorzugsweise für eine Zahl von 10 bis 100 steht; *** Polypropylsilsesquioxan **** Mischung aus Dimethiconol und Cyclodimethicon im Gewichtsverhältnis von Dimethiconol zu Cyclodimethicon 1:2 bis 1:10 | | | | | |

| | Formel 238 | Formel 239 | Formel 240 | Formel 241 | Formel 242 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Wasser | 40 bis 95 | 50 bis 92 | 60 bis 90 | 70 bis 88 | 86 |
| Misc | add 100 | add 100 | add 100 | add 100 | add 100 |

| | Formel 243 | Formel 244 | Formel 245 | Formel 246 | Formel 247 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Wasser | 40 bis 95 | 50 bis 92 | 60 bis 90 | 70 bis 88 | 86 |
| Misc | add 100 | add 100 | add 100 | add 100 | add 100 |

| | Formel 248 | Formel 249 | Formel 250 | Formel 251 | Formel 252 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Fettalkohol | 0,1 bis 10 | 0,2 bis 8,0 | 0,3 bis 6,0 | 0,6 bis 5,0 | 0,5 |
| Wasser | 40 bis 95 | 50 bis 92 | 60 bis 90 | 70 bis 88 | 86 |
| Misc | add 100 | add 100 | add 100 | add 100 | add 100 |

| | Formel 253 | Formel 254 | Formel 255 | Formel 256 | Formel 257 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Natürliches Öl | 0,1 bis 10 | 0,2 bis 8,0 | 0,3 bis 6,0 | 0,6 bis 5,0 | 3,0 |
| Wasser | 40 bis 95 | 50 bis 92 | 60 bis 90 | 70 bis 88 | 86 |
| Misc | add 100 | add 100 | add 100 | add 100 | add 100 |

| | Formel 258 | Formel 259 | Formel 260 | Formel 261 | Formel 262 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Esteröl | 0,1 bis 10 | 0,2 bis 8,0 | 0,3 bis 6,0 | 0,6 bis 5,0 | 1,0 |
| Wasser | 40 bis 95 | 50 bis 92 | 60 bis 90 | 70 bis 88 | 86 |
| Misc | add 100 | add 100 | add 100 | add 100 | add 100 |

| | Formel 263 | Formel 264 | Formel 265 | Formel 266 | Formel 267 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Fettalkohol | 0,1 bis 10 | 0,2 bis 8,0 | 0,3 bis 6,0 | 0,6 bis 5,0 | 0,5 |
| Wasser | 40 bis 95 | 50 bis 92 | 60 bis 90 | 70 bis 88 | 86 |
| Misc | add 100 | add 100 | add 100 | add 100 | add 100 |

| | Formel 268 | Formel 269 | Formel 270 | Formel 271 | Formel 272 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Natürliches Öl | 0,1 bis 10 | 0,2 bis 8,0 | 0,3 bis 6,0 | 0,6 bis 5,0 | 3,0 |
| Wasser | 40 bis 95 | 50 bis 92 | 60 bis 90 | 70 bis 88 | 86 |
| Misc | add 100 | add 100 | add 100 | add 100 | add 100 |

| | Formel 273 | Formel 274 | Formel 275 | Formel 276 | Formel 277 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Esteröl | 0,1 bis 10 | 0,2 bis 8,0 | 0,3 bis 6,0 | 0,6 bis 5,0 | 1,0 |
| Wasser | 40 bis 95 | 50 bis 92 | 60 bis 90 | 70 bis 88 | 86 |
| Misc | add 100 | add 100 | add 100 | add 100 | add 100 |

| | Formel 278 | Formel 279 | Formel 280 | Formel 281 | Formel 282 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Fettalkohol | 0,1 bis 10 | 0,2 bis 8,0 | 0,3 bis 6,0 | 0,6 bis 5,0 | 0,5 |
| Natürliches Öl | 0,1 bis 10 | 0,2 bis 8,0 | 0,3 bis 6,0 | 0,6 bis 5,0 | 3,0 |
| Wasser | 40 bis 95 | 50 bis 92 | 60 bis 90 | 70 bis 88 | 86 |
| Misc | add 100 | add 100 | add 100 | add 100 | add 100 |

| | Formel 283 | Formel 284 | Formel 285 | Formel 286 | Formel 287 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Fettalkohol | 0,1 bis 10 | 0,2 bis 8,0 | 0,3 bis 6,0 | 0,6 bis 5,0 | 0,5 |
| Esteröl | 0,1 bis 10 | 0,2 bis 8,0 | 0,3 bis 6,0 | 0,6 bis 5,0 | 1,0 |
| Wasser | 40 bis 95 | 50 bis 92 | 60 bis 90 | 70 bis 88 | 86 |
| Misc | add 100 | add 100 | add 100 | add 100 | add 100 |

| | Formel 288 | Formel 289 | Formel 290 | Formel 291 | Formel 292 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Natürliches Öl | 0,1 bis 10 | 0,2 bis 8,0 | 0,3 bis 6,0 | 0,6 bis 5,0 | 3,0 |
| Esteröl | 0,1 bis 10 | 0,2 bis 8,0 | 0,3 bis 6,0 | 0,6 bis 5,0 | 1,0 |
| Wasser | 40 bis 95 | 50 bis 92 | 60 bis 90 | 70 bis 88 | 86 |
| Misc | add 100 | add 100 | add 100 | add 100 | add 100 |

| | | | | | |
|---|---|---|---|---|---|
| * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. ** polymere quartäre Ammoniumverbindung, vorzugsweise Homo- oder Copolymere eines Methacryloyloxyethyltrimethylammoniumhalogenids, besonders bevorzugt Poly(methacryloyloxyethyltrimethylammoniumchlorid) oder Acrylamid-Methacryloyloxyethyltrimethylammoniumchlorid-Copolymer *** monomere quartäre Ammoniumverbindung | | | | | |

Die Zusammensetzung einiger bevorzugter kosmetischer Mittel, die einen Silikongehalt unterhalb 1,0 Gew.-%, vorzugsweise unterhalb 0,5 Gew.-% aufweisen, besonders bevorzugt jedoch kein Silikon enthalten, kann den folgenden Tabellen entnommen werden (Angaben in Gew.-% bezogen auf das Gesamtgewicht des kosmetischen Mittels sofern nicht anders angegeben).

| | Formel 293 | Formel 294 | Formel 295 | Formel 296 | Formel 297 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Wasser | 40 bis 95 | 50 bis 92 | 60 bis 90 | 70 bis 88 | 86 |
| Misc | add 100 | add 100 | add 100 | add 100 | add 100 |

| | Formel 298 | Formel 299 | Formel 300 | Formel 301 | Formel 302 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Wasser | 40 bis 95 | 50 bis 92 | 60 bis 90 | 70 bis 88 | 86 |
| Misc | add 100 | add 100 | add 100 | add 100 | add 100 |

| | Formel 303 | Formel 304 | Formel 305 | Formel 306 | Formel 307 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Fettalkohol | 0,1 bis 10 | 0,2 bis 8,0 | 0,3 bis 6,0 | 0,6 bis 5,0 | 0,5 |
| Wasser | 40 bis 95 | 50 bis 92 | 60 bis 90 | 70 bis 88 | 86 |
| Misc | add 100 | add 100 | add 100 | add 100 | add 100 |

| | Formel 308 | Formel 309 | Formel 310 | Formel 311 | Formel 312 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Natürliches Öl | 0,1 bis 10 | 0,2 bis 8,0 | 0,3 bis 6,0 | 0,6 bis 5,0 | 3,0 |
| Wasser | 40 bis 95 | 50 bis 92 | 60 bis 90 | 70 bis 88 | 86 |
| Misc | add 100 | add 100 | add 100 | add 100 | add 100 |

| | Formel 313 | Formel 314 | Formel 315 | Formel 316 | Formel 317 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Esteröl | 0,1 bis 10 | 0,2 bis 8,0 | 0,3 bis 6,0 | 0,6 bis 5,0 | 1,0 |
| Wasser | 40 bis 95 | 50 bis 92 | 60 bis 90 | 70 bis 88 | 86 |
| Misc | add 100 | add 100 | add 100 | add 100 | add 100 |

| | Formel 318 | Formel 319 | Formel 320 | Formel 321 | Formel 322 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Fettalkohol | 0,1 bis 10 | 0,2 bis 8,0 | 0,3 bis 6,0 | 0,6 bis 5,0 | 0,5 |
| Wasser | 40 bis 95 | 50 bis 92 | 60 bis 90 | 70 bis 88 | 86 |
| Misc | add 100 | add 100 | add 100 | add 100 | add 100 |

| | Formel 323 | Formel 324 | Formel 325 | Formel 326 | Formel 327 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Natürliches Öl | 0,1 bis 10 | 0,2 bis 8,0 | 0,3 bis 6,0 | 0,6 bis 5,0 | 3,0 |
| Wasser | 40 bis 95 | 50 bis 92 | 60 bis 90 | 70 bis 88 | 86 |
| Misc | add 100 | add 100 | add 100 | add 100 | add 100 |

| | Formel 328 | Formel 329 | Formel 330 | Formel 331 | Formel 332 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Esteröl | 0,1 bis 10 | 0,2 bis 8,0 | 0,3 bis 6,0 | 0,6 bis 5,0 | 1,0 |
| Wasser | 40 bis 95 | 50 bis 92 | 60 bis 90 | 70 bis 88 | 86 |
| Misc | add 100 | add 100 | add 100 | add 100 | add 100 |

| | Formel 333 | Formel 334 | Formel 335 | Formel 336 | Formel 337 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Fettalkohol | 0,1 bis 10 | 0,2 bis 8,0 | 0,3 bis 6,0 | 0,6 bis 5,0 | 0,5 |
| Natürliches Öl | 0,1 bis 10 | 0,2 bis 8,0 | 0,3 bis 6,0 | 0,6 bis 5,0 | 3,0 |
| Wasser | 40 bis 95 | 50 bis 92 | 60 bis 90 | 70 bis 88 | 86 |
| Misc | add 100 | add 100 | add 100 | add 100 | add 100 |

| | Formel 338 | Formel 339 | Formel 340 | Formel 341 | Formel 342 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Fettalkohol | 0,1 bis 10 | 0,2 bis 8,0 | 0,3 bis 6,0 | 0,6 bis 5,0 | 0,5 |
| Esteröl | 0,1 bis 10 | 0,2 bis 8,0 | 0,3 bis 6,0 | 0,6 bis 5,0 | 1,0 |
| Wasser | 40 bis 95 | 50 bis 92 | 60 bis 90 | 70 bis 88 | 86 |
| Misc | add 100 | add 100 | add 100 | add 100 | add 100 |

| | Formel 343 | Formel 344 | Formel 345 | Formel 346 | Formel 347 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| Monomer *** | 0,1 bis 10 | 0,25 bis 8,0 | 0,3 bis 6,0 | 0,6 bis 5,0 | 0,8 |
| Natürliches Öl | 0,1 bis 10 | 0,2 bis 8,0 | 0,3 bis 6,0 | 0,6 bis 5,0 | 3,0 |
| Esteröl | 0,1 bis 10 | 0,2 bis 8,0 | 0,3 bis 6,0 | 0,6 bis 5,0 | 1,0 |
| Wasser | 40 bis 95 | 50 bis 92 | 60 bis 90 | 70 bis 88 | 86 |
| Misc | add 100 | add 100 | add 100 | add 100 | add 100 |

| | Formel 348 | Formel 349 | Formel 350 | Formel 351 | Formel 352 |
|---|---|---|---|---|---|
| Isoparaffin * | 0,1 bis 9,0 | 0,2 bis 8,0 | 0,5 bis 6,0 | 1,0 bis 4,0 | 2,2 |
| Polymer ** | 0,1 bis 10 | 0,2 bis 8,0 | 0,5 bis 5,0 | 0,5 bis 5,0 | 1,2 |
| quartäres Imidazolin | 0,1 bis 10 | 0,25 bis 8,0 | 0,3 bis 6,0 | 0,6 bis 5,0 | 0,8 |
| C16-18 Fettalkohol | 0,1 bis 10 | 0,2 bis 8,0 | 0,3 bis 6,0 | 0,6 bis 5,0 | 0,5 |
| Aprikosenkernöl | 0,1 bis 10 | 0,2 bis 8,0 | 0,3 bis 6,0 | 0,6 bis 5,0 | 3,0 |
| Esteröl | 0,1 bis 10 | 0,2 bis 8,0 | 0,3 bis 6,0 | 0,6 bis 5,0 | 1,0 |
| Panthenol | 0,02 bis 3,0 | 0,05 bis 2,0 | 0,1 bis 1,5 | 0,2 bis 1,0 | 0,25 |
| Glycerin | 0,1 bis 5,0 | 0,2 bis 4,0 | 0,3 bis 3,0 | 0,4 bis 2,0 | 0,9 |
| Wasser | 40 bis 95 | 50 bis 92 | 60 bis 90 | 70 bis 88 | 86 |
| Misc | add 100 | add 100 | add 100 | add 100 | add 100 |

| | Formel 353 | Formel 354 | Formel 355 | Formel 356 | Formel 357 |
|---|---|---|---|---|---|
| C10-C13 Isoparaffin | 0,7 | 3,3 | 1,3 | 1,8 | 2,2 |
| Polyquaternium-37 | 2,4 | 0,8 | 1,7 | 1,1 | 1,2 |
| Quaternium-87 | 0,5 | 0,8 | 1,2 | 0,8 | 0,8 |
| Isopropylmyristat | 0,8 | 1,2 | 1,5 | 0,4 | 1,0 |
| Glycerylstearat | 0,2 | 0,5 | 1,4 | 2,1 | 0,6 |
| C16-18 Fettalkohol | 4,0 | 2,7 | -- | 1,3 | 0,5 |
| Aprikosenkernöl | -- | 1,4 | 2,3 | -- | 3,0 |
| Arganöl | 3,0 | 1,4 | -- | 2,4 | -- |
| Olivenöl | 1,0 | -- | 1,8 | 0,4 | 0,3 |
| Panthenol | -- | 0,1 | 0,4 | 0,1 | 0,25 |
| Glycerin | -- | 0,4 | 1,2 | 1,5 | 0,9 |
| Proteinhydrolysat | 0,08 | 0,12 | 0,2 | -- | 0,2 |
| Wasser | 83 | 85 | 78 | 84 | 86 |
| Misc | add 100 | add 100 | add 100 | add 100 | add 100 |

| | Formel 358 | Formel 359 | Formel 360 | Formel 361 | Formel 362 |
|---|---|---|---|---|---|
| Silikonöl ** | 20 bis 90 | 25 bis 88 | 30 bis 85 | 35 | 80 |
| Isoparaffin *, Parfüm | add 100 | add 100 | add 100 | add 100 | add 100 |

| | Formel 363 | Formel 364 | Formel 365 | Formel 366 | Formel 367 |
|---|---|---|---|---|---|
| Isoparaffin * | 10 bis 70 | 12 bis 65 | 15 bis 60 | 40 | 14 |
| natürliches Öl** | 0,1 bis 10 | 0,2 bis 8,0 | 0,4 bis 5,0 | 0,2 | 0,6 |
| Isoparaffin *, Parfüm | add 100 | add 100 | add 100 | add 100 | add 100 |

| | Formel 368 | Formel 369 | Formel 370 | Formel 371 | Formel 372 |
|---|---|---|---|---|---|
| Silikonöl | 25 bis 88 | 25 bis 88 | 30 bis 85 | 35 | 80 |
| natürliches Öl | 0,1 bis 10 | 0,2 bis 8,0 | 0,4 bis 5,0 | 0,2 | 0,6 |
| Isoparaffin *, Parfüm | add 100 | add 100 | add 100 | add 100 | add 100 |

| | Formel 373 | Formel 374 | Formel 375 | Formel 376 | Formel 377 |
|---|---|---|---|---|---|
| Silikonöl ** | 25 bis 88 | 25 bis 88 | 30 bis 85 | 35 | 80 |
| natürliches Öl | 0,1 bis 10 | 0,2 bis 8,0 | 0,4 bis 5,0 | 0,2 | 0,6 |
| Isoparaffin *, Parfüm | add 100 | add 100 | add 100 | add 100 | add 100 |

| | Formel 378 | Formel 379 | Formel 380 | Formel 381 | Formel 382 |
|---|---|---|---|---|---|
| Esteröl | 0,1 bis 30 | 0,2 bis 27 | 0,4 bis 24 | 20 | 0,6 |
| Isoparaffin *, Parfüm | add 100 | add 100 | add 100 | add 100 | add 100 |

| | Formel 383 | Formel 384 | Formel 385 | Formel 386 | Formel 387 |
|---|---|---|---|---|---|
| Isopropylmyristat | 0,1 bis 30 | 0,2 bis 27 | 0,4 bis 24 | 20 | 0,6 |
| Isoparaffin *, Parfüm | add 100 | add 100 | add 100 | add 100 | add 100 |

| | Formel 388 | Formel 389 | Formel 390 | Formel 391 | Formel 392 |
|---|---|---|---|---|---|
| Silikonöl | 25 bis 88 | 25 bis 88 | 30 bis 85 | 35 | 80 |
| Esteröl | 0,1 bis 30 | 0,2 bis 27 | 0,4 bis 24 | 20 | 0,6 |
| Isoparaffin *, Parfüm | add 100 | add 100 | add 100 | add 100 | add 100 |

| | Formel 393 | Formel 394 | Formel 395 | Formel 396 | Formel 397 |
|---|---|---|---|---|---|
| Silikonöl | 25 bis 88 | 25 bis 88 | 30 bis 85 | 35 | 80 |
| Isopropylmyristat | 0,1 bis 30 | 0,2 bis 27 | 0,4 bis 24 | 20 | 0,6 |
| Isoparaffin *, Parfüm | add 100 | add 100 | add 100 | add 100 | add 100 |

| | Formel 398 | Formel 399 | Formel 400 | Formel 401 | Formel 402 |
|---|---|---|---|---|---|
| Silikonöl ** | 25 bis 88 | 25 bis 88 | 30 bis 85 | 35 | 80 |
| Esteröl | 0,1 bis 30 | 0,2 bis 27 | 0,4 bis 24 | 20 | 0,6 |
| Isoparaffin *, Parfüm | add 100 | add 100 | add 100 | add 100 | add 100 |

| | Formel 403 | Formel 404 | Formel 405 | Formel 406 | Formel 407 |
|---|---|---|---|---|---|
| Silikonöl ** | 25 bis 88 | 25 bis 88 | 30 bis 85 | 35 | 80 |
| Isopropylmyristat | 0,1 bis 30 | 0,2 bis 27 | 0,4 bis 24 | 20 | 0,6 |
| Isoparaffin *, Parfüm | add 100 | add 100 | add 100 | add 100 | add 100 |

| | Formel 408 | Formel 409 | Formel 410 | Formel 411 | Formel 412 |
|---|---|---|---|---|---|
| Silikonöl | 25 bis 88 | 25 bis 88 | 30 bis 85 | 35 | 80 |
| Natürliches Öl | 0,1 bis 10 | 0,2 bis 8,0 | 0,4 bis 5,0 | 0,2 | 0,6 |
| Esteröl | 0,1 bis 30 | 0,2 bis 27 | 0,4 bis 24 | 20 | 0,6 |
| Isoparaffin *, Parfüm | add 100 | add 100 | add 100 | add 100 | add 100 |

| | Formel 413 | Formel 414 | Formel 415 | Formel 416 | Formel 417 |
|---|---|---|---|---|---|
| Silikonöl | 25 bis 88 | 25 bis 88 | 30 bis 85 | 35 | 80 |
| Natürliches Öl | 0,1 bis 10 | 0,2 bis 8,0 | 0,4 bis 5,0 | 0,2 | 0,6 |
| Isopropylmyristat | 0,1 bis 30 | 0,2 bis 27 | 0,4 bis 24 | 20 | 0,6 |
| Isoparaffin *, Parfüm | add 100 | add 100 | add 100 | add 100 | add 100 |

| | Formel 418 | Formel 419 | Formel 420 | Formel 421 | Formel 422 |
|---|---|---|---|---|---|
| Silikonöl ** | 25 bis 88 | 25 bis 88 | 30 bis 85 | 35 | 80 |
| Natürliches Öl | 0,1 bis 10 | 0,2 bis 8,0 | 0,4 bis 5,0 | 0,2 | 0,6 |
| Esteröl | 0,1 bis 30 | 0,2 bis 27 | 0,4 bis 24 | 20 | 0,6 |
| Isoparaffin *, Parfüm | add 100 | add 100 | add 100 | add 100 | add 100 |

| | Formel 423 | Formel 424 | Formel 425 | Formel 426 | Formel 427 |
|---|---|---|---|---|---|
| Silikonöl ** | 25 bis 88 | 25 bis 88 | 30 bis 85 | 35 | 80 |
| Natürliches Öl | 0,1 bis 10 | 0,2 bis 8,0 | 0,4 bis 5,0 | 0,2 | 0,6 |
| Isopropylmyristat | 0,1 bis 30 | 0,2 bis 27 | 0,4 bis 24 | 20 | 0,6 |
| Isoparaffin *, Parfüm | add 100 | add 100 | add 100 | add 100 | add 100 |

| | Formel 428 | Formel 429 | Formel 430 | Formel 431 | Formel 432 |
|---|---|---|---|---|---|
| Silikonöl | 25 bis 88 | 25 bis 88 | 30 bis 85 | 35 | 80 |
| Macadamianussöl | 0,1 bis 3,0 | 0,1 bis 2,0 | 0,1 bis 1,0 | 0,2 | 0,15 |
| Arganöl | 0,1 bis 3,0 | 0,1 bis 2,0 | 0,1 bis 1,0 | .. | 0,15 |
| Aprikosenkernöl | 0,1 bis 3,0 | 0,1 bis 2,0 | 0,1 bis 1,0 | .. | 0,15 |
| Olivenöl | 0,1 bis 3,0 | 0,1 bis 2,0 | 0,1 bis 1,0 | .. | 0,15 |
| Esteröl | 0,1 bis 30 | 0,2 bis 27 | 0,4 bis 24 | 20 | 0,6 |
| Isoparaffin *, Parfüm | add 100 | add 100 | add 100 | add 100 | add 100 |

| | Formel 433 | Formel 434 | Formel 435 | Formel 436 | Formel 437 |
|---|---|---|---|---|---|
| Silikonöl | 25 bis 88 | 25 bis 88 | 30 bis 85 | 35 | 80 |
| Macadamianussöl | 0,1 bis 3,0 | 0,1 bis 2,0 | 0,1 bis 1,0 | 0,2 | 0,15 |
| Arganöl | 0,1 bis 3,0 | 0,1 bis 2,0 | 0,1 bis 1,0 | .. | 0,15 |
| Aprikosenkernöl | 0,1 bis 3,0 | 0,1 bis 2,0 | 0,1 bis 1,0 | .. | 0,15 |
| Olivenöl | 0,1 bis 3,0 | 0,1 bis 2,0 | 0,1 bis 1,0 | .. | 0,15 |
| Isopropylmyristat | 0,1 bis 30 | 0,2 bis 27 | 0,4 bis 24 | 20 | 0,6 |
| Isoparaffin *, Parfüm | add 100 | add 100 | add 100 | add 100 | add 100 |

| | Formel 438 | Formel 439 | Formel 440 | Formel 441 | Formel 442 |
|---|---|---|---|---|---|
| Silikonöl ** | 25 bis 88 | 25 bis 88 | 30 bis 85 | 35 | 80 |
| Macadamianussöl | 0,1 bis 3,0 | 0,1 bis 2,0 | 0,1 bis 1,0 | 0,2 | 0,15 |
| Arganöl | 0,1 bis 3,0 | 0,1 bis 2,0 | 0,1 bis 1,0 | | 0,15 |
| Aprikosenkernöl | 0,1 bis 3,0 | 0,1 bis 2,0 | 0,1 bis 1,0 | | 0,15 |
| Olivenöl | 0,1 bis 3,0 | 0,1 bis 2,0 | 0,1 bis 1,0 | .. | 0,15 |
| Esteröl | 0,1 bis 30 | 0,2 bis 27 | 0,4 bis 24 | 20 | 0,6 |
| Isoparaffin *, Parfüm | add 100 | add 100 | add 100 | add 100 | add 100 |

| | Formel 443 | Formel 444 | Formel 445 | Formel 446 | Formel 447 |
|---|---|---|---|---|---|
| Silikonöl ** | 25 bis 88 | 25 bis 88 | 30 bis 85 | 35 | 80 |
| Macadamianussöl | 0,1 bis 3,0 | 0,1 bis 2,0 | 0,1 bis 1,0 | 0,2 | 0,15 |
| Arganöl | 0,1 bis 3,0 | 0,1 bis 2,0 | 0,1 bis 1,0 | .. | 0,15 |
| Aprikosenkernöl | 0,1 bis 3,0 | 0,1 bis 2,0 | 0,1 bis 1,0 | .. | 0,15 |
| Olivenöl | 0,1 bis 3,0 | 0,1 bis 2,0 | 0,1 bis 1,0 | .. | 0,15 |
| Isopropylmyristat | 0,1 bis 30 | 0,2 bis 27 | 0,4 bis 24 | 20 | 0,6 |
| Isoparaffin *, Parfüm | add 100 | add 100 | add 100 | add 100 | add 100 |
| * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. ** polymere quartäre Ammoniumverbindung, vorzugsweise Homo- oder Copolymere eines Methacryloyloxyethyltrimethylammoniumhalogenids, besonders bevorzugt Poly(methacryloyloxyethyltrimethylammoniumchlorid) oder Acrylamid-Methacryloyloxyethyltrimethylammoniumchlorid-Copolymer *** monomere quartäre Ammoniumverbindung * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. ** Poly(Methacryloyloxyethyltrimethylammoniumchlorid) oder Acrylamid-Methacryloyloxyethyltrimethylammoniumchlorid-Copolymer * Isoparaffingemisch, umfassend mindestens ein Isoparaffin aus der Gruppe Isododecan und Isotridecan, wobei der Gewichtsanteil des Isodedecans und Isotridecans am Gesamtgewicht aller in dem kosmetischen Mittel eingesetzten Isoparaffine vorzugsweise mehr als 60 Gew.-%, bevorzugt mehr als 70 Gew.-%, besonders bevorzugt mehr als 80 Gew.-% und insbesondere mehr als 90 Gew.-% beträgt. ** Mischung aus Dimethiconol und Cyclodimethicon im Gewichtsverhältnis von Dimethiconol zu Cyclodimethicon 1:2 bis 1:10 | | | | | |

Wie eingangs ausgeführt, eignen sich die erfindungsgemäßen Mittel insbesondere zur Haarpflege. Ein weiterer Gegenstand der vorliegenden Anmeldung ist daher ein Verfahren zur Behandlung keratinischer Fasern dadurch gekennzeichnet, dass ein erfindungsgemäßes kosmetisches Mittel auf die getrockneten und/oder Handtuchfeuchten keratinischen Fasern aufgetragen wird.

Das erfindungsgemäße Haarbehandlungsmittel wird bevorzugt unmittelbar vor, während oder nach einer oxidativen oder tensidischen Haarbehandlung verwendet. Als unmittelbar vor der oxidativen oder tensidischen Haarbehandlung versteht sich im Sinne der Erfindung eine Anwendung, an die sich direkt die oxidative oder tensidische Haarbehandlung anschließt, wobei das erfindungsgemäße Haarbehandlungsmittel zuvor vom Haar gespült oder bevorzugt auf dem Haar belassen wurde und das Haar bevorzugt noch nass ist.

Als nach der oxidative oder tensidischen Haarbehandlung versteht sich im Sinne der Erfindung eine Anwendung, welche sich entweder direkt an die oxidative oder tensidische Haarbehandlung anschließt, wobei das erfindungsgemäße Haarbehandlungsmittel nach dem Abspülen des oxidativ oder tensidisch wirkenden Mittels auf das bevorzugt noch nasse, handtuchtrockene Haar aufgetragen wird, oder erst nach mehreren Stunden oder Tagen auf das trockene oder nasse Haar aufgetragen wird. In beiden Fällen kann das erfindungsgemäße Haarbehandlungsmittel hierbei nach einer Einwirkzeit von wenigen Sekunden bis hin zu 45 Minuten wieder ausgespült werden oder vollständig auf dem Haar verbleiben.

Die Wirkung des erfindungsgemäßen Haarbehandlungsmittels entfaltet sich sogar während der oxidativen oder tensidischen Haarbehandlung und hält überraschenderweise auch nach intensivem Auswaschen des erfindungsgemäßen Haarbehandlungsmittels an.

Ein weiterer Gegentand der vorliegenden Anmeldung ist die Verwendung eines erfindungsgemäßen kosmetischen Mittels zur Pflege keratinischer Fasern. Beansprucht wird dabei insbesondere die Verwendung eines erfindungsgemäßen kosmetischen Mittels
- zur Verbesserung der Nass- und Trockenkämmbarkeiten keratinischer Fasern,
- zur Verbesserung des Glanzes keratinischer Fasern,
- zur Verbesserung des Feuchtehaushaltes keratinischer Fasern,
- zum Schutz der keratinischen Fasern vor oxidativen Schädigungen, insbesondere im Zuge oxidativer Haarbehandlungen,
- zum Verhindern des Nachfettens keratinischer Fasern,
- zur Erhöhung der Waschbeständigkeit gefärbter keratinischer Fasern,
- zur Erzielung eines erhöhten Frisurenhaltes über mindestens 48 h, insbesondere 24 h hinweg,
- zur Verbesserung der sogenannten curl-retention keratinischer Fasern,
- zur Verbesserung des Griffs keratinischer Fasern,
- zur Verbesserung der Regeneration keratinischer Fasern.

Unter Kämmbarkeit versteht sich erfindungsgemäß sowohl die Kämmbarkeit der nassen Faser, als auch die Kämmbarkeit der trockenen Faser. Als Maß für die Kämmbarkeit dient die aufgewendete Kämmarbeit oder die aufgewendete Kraft während des Kämmvorganges eines Faserkollektivs. Die Messparameter können durch den Fachmann sensorisch beurteilt oder durch Messeinrichtungen quantifiziert werden.

Unter einer oxidativen Haarbehandlung wird erfindungsgemäß die Einwirkung eines oxidativen kosmetischen Mittels, enthaltend in einem kosmetischen Träger mindestens ein Oxidationsmittel, auf Haar definiert.

Als Griff definiert sich die Taktilität eines Faserkollektivs, wobei der Fachmann sensorisch die Parameter Fülle und Geschmeidigkeit des Kollektivs fühlt und bewertet.

### Beispiele

Alle Mengenangaben sind, soweit nicht anders vermerkt, Gewichtsteile. Die folgenden Rezepturen wurden unter Anwendung bekannter Herstellungsverfahren bereitgestellt

| **Haaröle** | Öl 1 | Öl 2 | Öl 3 | Öl 4 | Öl 5 | Öl 6 | Öl 7 |
|---|---|---|---|---|---|---|---|
| Cyclomethicone and Dimethicone | 35,0 | 50,0 | 80,0 | ---- | 50,0 | 50,0 | 80,0 |
| Isopropylmyristat | 20,0 | ---- | ---- | ---- | ---- | ---- | ---- |
| Macadamianussöl | 0,2 | 0,1 | 0,1 | 0,1 | ---- | ---- | ---- |
| Marulaöl | ---- | 0,1 | 0,1 | 0,1 | ---- | ---- | ---- |
| Arganöl | ---- | 0,1 | 0,1 | 0,1 | ---- | ---- | ---- |
| Aprikosenkernöl | ---- | 0,1 | 0,1 | 0,1 | ---- | ---- | ---- |
| Mandelöl | ---- | 0,1 | 0,1 | 0,1 | ---- | ---- | ---- |
| Olivenöl | ---- | 0,1 | 0,1 | 0,1 | ---- | ---- | ---- |
| Sesamöl | ---- | 0,1 | 0,1 | 0,1 | ---- | ---- | ---- |
| Silicone Quaternium-22 | ---- | ---- | ---- | ---- | ---- | 1,0 | ---- |
| Trimethylsiloxysilicate and Polypropylsilsesquioxane | ---- | ---- | ---- | ---- | ---- | ---- | 0,5 |
| C10-C13 Isoparaffin | Ad 100 | Ad 100 | Ad 100 | Ad 100 | 49,2 | 18,7 | 49,2 |
| Parfüm. Konservierung, Wasser, pH-Wert Einstellung | q.s. | q.s | q.s. | q.s. | q.s. | q.s. | q.s. |

| **Haarkuren** | Kur 1 | Kur 2 |
|---|---|---|
| Isopropylmyristat | 1,0 | 1,6 |
| Glycerinmonostearat | 0,5 | 0,7 |
| Quaternium-87 | 2,0 | 1,0 |
| Distearoylethyl Hydroxyethylmonium Methosulfate | 1,0 | 0,75 |
| Cetearylalcohol | 4,8 | 0,25 |
| Ceteareth-20 | 0,5 | ---- |
| Stearamidopropyldimethylamine | 0,8 | 0,7 |
| Polyquaternium-37 | 1,3 | 2,0 |
| Dimethicone and Dimethiconol | ---- | 0,5 |
| C10-C13 Isoparaffin | 2,5 | 1,0 |
| C10-C13 Isoparaffin | 0,5 | 0,5 |
| Glycerin | 0,8 | 1,0 |
| Aprikosenkernöl | ---- | 3,0 |
| Proteinhydrolysat | 0,15 | ---- |
| Panthenol | 0,2 | 0,3 |
| Parfüm. Puffer, Konservierung, Wasser | Ad 100 | Ad 100 |

## Patentansprüche

1. Kosmetisches Mittel, enthaltend in einem kosmetisch akzeptablen Träger a) mindestens ein Isoparaffin aus der Gruppe Isodecan, Isoundecan, Isododecan, Isotridecan und Isotetradecan,
b) mindestens eine Polymer aus der Gruppe
b1) der Silikonpolymere mit quartärer Ammoniumfunktion
b2) der Silsesquioxane und/oder
c) mindestens eine polymere quartäre Ammoniumverbindung und/oder
d) mindestens einen Ölkörper.

2. Kosmetisches Mittel nach Anspruch 1, **dadurch gekennzeichnet, dass** das kosmetische Mittel mindestens ein Isoparaffin a) aus der Gruppe Isoundecan, Isododecan, Isotridecan enthält.

3. Kosmetisches Mittel nach einem der vorhergehenden Ansprüche, **dadurch gekennzeichnet, dass**
das Silikonpolymer b1) ausgewählt ist aus den Silikonpolymeren, die neben einer quartären Ammoniumfunktion weiterhin auch freie Hydroxylgruppen aufweisen.

4. Kosmetisches Mittel nach einem der vorgehenden Ansprüche, **dadurch gekennzeichnet, dass** der Gewichtsanteil des Silikonpolymers b1) am Gesamtgewicht des kosmetischen Mittels 0,05 bis 5,0 Gew.-%, vorzugsweise 0,1 bis 3,0 Gew.-%, besonders bevorzugt 0,2 bis 2,0 Gew.-%
und
insbesondere 0,5 bis 1,5 Gew.-% beträgt.

5. Kosmetisches Mittel nach einem der vorgehenden Ansprüche, **dadurch gekennzeichnet, dass** als Silikonpolymer b2) Polypropylsilsesquioxan eingesetzt wird.

6. Kosmetisches Mittel nach einem der vorgehenden Ansprüche, **dadurch gekennzeichnet, dass** der Gewichtsanteil des Silsesquioxans b2) am Gesamtgewicht des kosmetischen Mittels 0,05 bis 3,0 Gew.-%, vorzugsweise 0,1 bis 2,0 Gew.-%, besonders bevorzugt 0,15 bis 1,5 Gew.-% und insbesondere 0,2 bis 1,0 Gew.-% beträgt.

7. Kosmetisches Mittel nach einem der vorgehenden Ansprüche, **dadurch gekennzeichnet, dass**
das kosmetische Mittel mindestens eine polymere quartäre Ammoniumverbindung c) ausgewählt
aus der Gruppe der Polymere des Methacryloyloxyethyltrimethylammoniumchlorids enthält.

8. Kosmetisches Mittel nach einem der vorgehenden Ansprüche, **dadurch gekennzeichnet, dass** der Gewichtsanteil der polymeren quartären Ammoniumverbindung c) am Gesamtgewicht des kosmetischen Mittels 0,1 bis 10 Gew.-%, vorzugsweise 0,2 bis 8,0 Gew.-% und insbesondere 0,5 bis 5,0 Gew.-% beträgt.

9. Kosmetisches Mittel nach einem der vorgehenden Ansprüche, **dadurch gekennzeichnet, dass** das Gewichtsverhältnis von Isoparaffin a) zu polymerer quartärer Ammoniumverbindung c) zwischen 8:1 und 1:8, vorzugsweise zwischen 6:1 und 1:6 und insbesondere zwischen 4:1
und
1:4 beträgt.

10. Kosmetisches Mittel nach einem der vorgehenden Ansprüche, **dadurch gekennzeichnet, dass** es sich bei dem Ölkörper d) um ein Esteröl, vorzugsweise um Isopropylmyristat handelt.

11. Kosmetisches Mittel nach einem der vorgehenden Ansprüche, **dadurch gekennzeichnet, dass** es sich bei dem Ölkörper d) um ein natürliches Öl handelt.

12. Kosmetisches Mittel nach einem der vorgehenden Ansprüche, **dadurch gekennzeichnet, dass** das Gewichtsverhältnis von Isoparaffin a) zu Ölkörper d) 6:1 bis 1:6, vorzugsweise 4:1 bis 1:4 und insbesondere 2:1 bis 1:2 beträgt.

13. Kosmetisches Mittel nach einem der vorgehenden Ansprüche, **dadurch gekennzeichnet, dass** das kosmetische Mittel als weiteren Bestandteil mindestens eine monomere quartäre Ammoniumverbindung e) enthält.

14. Kosmetisches Mittel nach einem der vorgehenden Ansprüche, **dadurch gekennzeichnet, dass** es sich bei dem Ölkörper d) um ein natürliches Öl handelt.

15. Verwendung eines kosmetischen Mittels nach einem der Ansprüche 1 bis 14 zur Pflege keratinischer Fasern.
